# EUROPEAN PATENT SPECIFICATION

(11) **EP 1 845 938 B1**
(45) Date of publication and mention of the grant of the patent: **03.04.2019**
(21) Application number: 05852147.7
(22) Date of filing: 23.11.2005
(51) Int. Cl.: A61K 9/08, A61K 31/575, A61P 3/04, A61P 3/06

(54) **METHODS AND RELATED COMPOSITIONS FOR REDUCTION OF FAT AND SKIN TIGHTENING**
VERFAHREN UND DAMIT VERBUNDENE ZUSAMMENSETZUNGEN ZUR FETTREDUZIERUNG UND HAUTSTRAFFUNG
MÉTHODES ET COMPOSITIONS ASSOCIÉES PERMETTANT DE RÉDUIRE DES DÉPÔTS GRAISSEUX ET DE RETENDRE LA PEAU

(30) Priority: 08.02.2005 US 54171; 19.05.2005 US 134727
(43) Date of publication of application: 24.10.2007
(73) Proprietor: Los Angeles Biomedical Research Institute at Harbor-UCLA Medical Center, Torrance, CA 90250-2064 (US); The Regents of the University of California, Oakland, CA 94607 (US)
(72) Inventor: KOLODNEY, Michael S., Los Angeles, CA 90094 (US); ROTUNDA, Adam, M., Los Angeles, CA 90066 (US)
(74) Representative: Hoffmann Eitle
(86) International application number: PCT/US2005/042665
(87) International publication number: WO 2006/086038

(56) References cited:
- WO-A-2005/112942
- CA-C- 2 033 725
- US-A- 4 158 707
- US-A- 4 664 910
- US-A1- 2002 107 291
- ROTUNDA A M ET AL: "Detergent effects of sodium deoxycholate are a major feature of an injectable phosphatidylcholine formulation used for localized fat dissolution" DERMATOLOGIC SURGERY, ELSEVIER SCIENCE, NEW YORK, NY, US, vol. 30, no. 7, July 2004 (2004-07), pages 1001-1008, XP002331643 ISSN: 1076-0512
- DUNCAN ET AL: "Lipodissolve for subcutaneous fat reduction and skin retraction" AESTHETIC SURGERY JOURNAL, MOSBY-YEAR BOOK, ST. LOUIS, MO, US, vol. 25, no. 5, September 2005 (2005-09), pages 530-543, XP005119622 ISSN: 1090-820X

## Description

### BACKGROUND OF THE INVENTION

Surgical and non-surgical procedures for improving appearance have increased in prevalence as populations age and gain weight. Liposuction is a popular cosmetic surgery procedure and involves the surgical removal of fat deposits using suction and optionally assisted by solutions to assist in fat removal. Liposuction is a surgical procedure that removes fat through an incision in the skin through which a cannula is inserted. The cannula is connected to a suction source and the unwanted fat is aspirated through the cannula and discarded. Liposuction is performed under general or local anesthesia, depending on the amount and location of the fat to be removed. However, liposuction and other surgical methods of fat removal are associated with significant adverse events including temporary bruising, swelling, numbness, soreness and burning sensation, risk of infection, pigmentation changes, the formation of fat clots or blood clots which can migrate to the lungs and cause death, excessive fluid loss, which can lead to shock or fluid accumulation that must be drained, friction burns or other damage to the skin or nerves or perforation injury to the vital organs. Additionally, liposuction requires a recovery time of one to two weeks wherein the patient cannot work or perform certain daily activities. Moreover, because surgical procedures such as liposuction require local and occasionally general anesthesia, significant anesthesia-related risks are associated with surgical fat removal. Furthermore, liposuction and other drastic weight loss methods may result in loose skin.

Rotunda et al. (2004) Dermatol. Surg. 30(7): 1001-1008 describes results showing that detergent effects of sodium deoxycholate are a major feature of injectable phosphatidylcholine formulations used for localized fat dissolution.

US2002107291 discloses clear, injectable pharmaceutical compositions including propofol, a pharmaceutically acceptable salt of a bile acid and a lecithin.

US4158707 discloses aqueous vehicles for insoluble or sparingly soluble medicaments suitable for parenteral administration, including a micelle forming agent comprising cholic acid or a derivative thereof in combination with a lipoid.

CA2033725 discloses pharmaceutical and cosmetic preparations for topical administration, containing a salt of a cholanic acid, a lipid and optionally a pharmaceutical or cosmetic active substance.

US4664910 discloses a cosmetic composition suitable for application to the skin or hair as a powdered product to remove sebum and/or perspiration, comprising a cholanic acid derivative and a powder absorbent.

Therefore it would be desirable to have compositions and methods for removing localized fat accumulations that do not require surgery or prolonged recovery time as well as for tightening loose skin.

### SUMMARY OF THE INVENTION

The present invention provides compositions for therapeutic use in reducing subcutaneous fat deposits.

The compositions of the invention are in a solution. Preferably the solution is aqueous.

In one aspect, the present invention relates to a pharmaceutical solution for therapeutic use in the non-surgical removal of one or more localized fat deposits in a subject, wherein said solution is to be administered by subcutaneous injection, said solution consisting essentially of: between 0.05 and 1% w/w of a bile salt; and a pharmaceutical excipient; wherein the solution is free of phosphatidylcholine or phospholipids.

In some embodiments, a solution is in a container that contains up to 500 mL of solution. Such container can be a syringe or syringe-loadable container.

In some embodiments, a solution comprises a bile salt of a bile acid selected from the group consisting of: deoxycholic acid, cholic acid, chenodeoxycholic acid, 7-alpha-dehydroxylate chenodeoxycholic acid, lithocholic acid, ursodeoxycholic acid, dihydroxytaurin acid, trihydroxytaurine acid, and glycine conjugates of any of the above.

In some embodiments, a solution comprises a bile salt wherein the salt comprises a cation selected from the group consisting of sodium (Na⁺), potassium (K⁺), lithium (Li⁺), magnesium (Mg²⁺), calcium (Ca²⁺), barium (Ba²⁺), strontium (Sr²⁺), and ammonium (NH₄⁺).

Preferably, when the above solution comprises a bile salt, the bile salt is sodium deoxycholate.

In some embodiments, a solution comprises a bile salt that includes an alkali metal or an alkaline earth metal. Preferably an alkali metal is selected from the group consisting of alkali metal is sodium (Na⁺), potassium (K⁺), and lithium (Li⁺). Preferably, an alkaline earth metal is selected from the group consisting of magnesium (Mg²⁺), calcium (Ca²⁺), barium (Ba²⁺), and strontium (Sr²⁺).

The solutions include a therapeutically effective amount of the bile salt. Such therapeutically effective amounts are effective to reduce a subcutaneous fat deposit.

The bile salt in a solution of the invention is at a concentration of between 0.05 and 1% w/w.

Also disclosed are methods for reducing the appearance of a skin condition in a skin region of a subject. Such methods comprise the step of: administering locally to said skin region a composition comprising: (i) a skin-tightening effective amount of one or more pharmacologically active detergents, or bile acid(s) and/or bile salt(s), or deoxycholic acid or a salt thereof, or sodium deoxycholate, (ii) a pharmaceutical, veterinary, or cosmetic excipient, and (iii) optionally a lipid.

The administering step may involve delivering the compositions herein via a subcutaneous or transdermal injection.

The administering step may involve delivering the compositions herein via a dermal patch, a pump, or subdermal depot.

The administering step may involve delivering the compositions herein topically or subcutaneously.

The skin condition to be treated or ameliorated may be selected from the group consisting of: loose skin, skin aging, irregularities of the skin, and wrinkles.

The region of skin being treated may be under eye, under chin, under arm, buttock, cheek, brow, calf, back, thigh, ankle, or stomach.

The compositions used for reducing the appearance of a skin condition in a skin region may be formulated into a skin tightening solution.

Such skin tightening solution can further comprise a second therapeutic agent selected from the group consisting of: anti-microbial agents, vasoconstrictors, anti-thrombotic agents, anti-coagulation agents, suds-depressants, anti-inflammatory agents, analgesics, dispersion agents, anti-dispersion agents, penetration enhancers, steroids, tranquilizers, muscle relaxants, and anti-diarrhea agents.

The skin tightening solution may be in a container that contains up to 500 mL of solution. Such container can be a syringe or syringe-loadable container.

The skin tightening solution may comprise a bile acid selected from the group consisting of: deoxycholic acid, cholic acid, chenodeoxycholic acid, 7-alpha-dehydroxylate chenodeoxycholic acid, lithocholic acid, ursodeoxycholic acid, dihydroxytaurin acid, trihydroxytaurine acid, and glycine conjugates of any of the above.

The skin tightening solution may comprise a bile salt wherein the salt comprises a cation selected from the group consisting of sodium (Na⁺), potassium (K⁺), lithium (Li⁺), magnesium (Mg²⁺), calcium (Ca²⁺), barium (Ba²⁺), strontium (Sr²⁺), and ammonium (NH₄⁺).

Preferably, when the above solution comprises a bile salt, the bile salt is sodium deoxycholate.

The skin tightening solution may comprise a bile salt that includes an alkali metal or an alkaline earth metal. Preferably an alkali metal is selected from the group consisting of alkali metal is sodium (Na⁺), potassium (K⁺), and lithium (Li⁺). Preferably, an alkaline earth metal is selected from the group consisting of magnesium (Mg²⁺), calcium (Ca²⁺), barium (Ba²⁺), and strontium (Sr²⁺).

The skin tightening solution may include a therapeutically effective amount of the pharmacological detergents (e.g., bile acid(s) and/or bile salt(s)).

Such therapeutically effective amount is effective to tighten a loose skin region.

The skin tightening solution may comprise one or more pharmacologically active detergents (e.g., bile acid(s) and/or bile salt(s), such as sodium deoxycholate) at a concentration of about 0.001 to 10, 0.01 to 5, or 0.1 to 2% w/w, w/v, or v/v. Preferably, the one or more pharmacologically active detergents in the skin tightening solution is at a concentration of about 0.1-5 % w/w, or more preferably about 1% w/w.

The skin tightening solution may comprise up to 100, 50, 20, 10, 5, 2, 1, 0.5, 0.2, 0.05, 0.02, or 0.01 grams of the one or more detergents, bile acids and/or bile salts, deoxycholic acid or salts thereof or sodium deoxycholate.

The skin tightening solution may include no lipids, phospholipids, or phosphatidylcholine. The solutions may include up to 5% w/w, w/v, or v/v lipids, phospholipids, or phosphatidylcholine.

Also disclosed are methods for reducing a subcutaneous fat deposit in a subject. Such methods comprise the step of administering locally to a subcutaneous fat deposit in the subject a composition comprising: (i) a fat-dissolving effective amount of one or more pharmacologically active detergents, or bile acid(s) and/or bile salt(s), or deoxycholic acid or a salt thereof, or sodium deoxycholate; (ii) a pharmaceutical, veterinary, or cosmetic excipient; and (iii) optionally a lipid, wherein the ratio of the lipid and bile acid or bile salt is up to 1% w/w and wherein the composition does not include lipase or colipase.

The fat deposit may be associated with a condition selected from the group consisting of obesity, fat redistribution syndrome, eyelid fat herniation, lipomas, Dercum's disease, lipodystrophy, buffalo hump lipodystrophy, dorsocervical fat, visceral adiposity, breast enlargement, hyperadiposity, diffused body fat around trunk and arms, and fat deposits associated with cellulite.

The detergent may comprise a bile acid selected from the group consisting of deoxycholic acid, cholic acid, chenodeoxycholic acid, 7-alpha-dehydroxylate chenodeoxycholic acid, lithocholic acid, ursodeoxycholic acid, dihydroxytaurin acid, trihydroxytaurine acid, and glycine conjugates of any of the above.

The detergent may comprise a bile salt that includes a cation selected from the group consisting of sodium (Na⁺), potassium (K⁺), lithium (Li⁺), magnesium (Mg²⁺), calcium (Ca²⁺), barium (Ba²⁺), strontium (Sr²⁺), and ammonium (NH₄⁺).

The detergent may comprise a bile salt with a cation that is an alkali metal or an alkaline earth metal. Preferably, the alkali metal is sodium (Na⁺), potassium (K⁺), or lithium (Li⁺) and the alkaline earth metal is magnesium (Mg²⁺), calcium (Ca²⁺), barium (Ba²⁺), or strontium (Sr²⁺). More preferably, the bile salt is sodium deoxycholate.

The above method does not include performing surgery on said subject.

The administration step may involve administering locally (e.g., subcutaneously or subdermally) to a region under eye, under chin, under arm, buttock, calf, back, thigh, or stomach of said subject. The administration can be made by a subcutaneous or transdermal injection.

The subject to be treated by the compositions herein can be a human or a domesticated animal such as a cat, dog, or horse. In some embodiments, the subject to be treated is a human HIV patient. Such patient can be undergoing a HIV protease treatment or experiencing or susceptible to experiencing lipodystrophy.

Preferably, the compositions once administered are not removed from the subject. Furthermore, the compositions herein are preferably administered without a surgical procedure (e.g., liposuction).

The fat dissolving solution may comprise up to 100, 50, 20, 10, 5, 2, 1, 0.5, 0.2, 0.05, 0.02, or 0.01 grams of the one or more detergents, bile acids and/or bile salts, deoxycholic acid or salts thereof or sodium deoxycholate.

Also disclosed is a syringe loadable container comprising: (i) a fat-dissolving or skin-tightening effective amount of one or more pharmacologically active detergents, or bile acid(s) and/or bile salt(s), or deoxycholic acid or a salt thereof, or sodium deoxycholate; (ii) a pharmaceutical, veterinary, or cosmetic excipient; and (iii) optionally a lipid wherein the ratio of said bile acid or bile salt to said lipid is greater than 1 % w/w and wherein the solution does not contain lipase or colipase.

Preferably, the one or more detergents herein comprise sodium deoxycholate.

Preferably the container contains up to 500, 200, 100, 50, 20, 10, 5, 2, or 1 mL of a solution that is sterile.

In some embodiments, the solution comprises an organic solvent, or more preferably benzyl alcohol.

In some embodiments, the solution comprises 0.1 to 1% w/w bile salt such as sodium deoxycholate. In some embodiments, the solution comprises sodium deoxycholate in a concentration of between 0.05% and 0.5%. In some embodiments, the solution comprises sodium deoxycholate in a concentration of between 0.1% and 0.5%, such as 0.1% or 0.5%.

### BRIEF DESCRIPTION OF THE DRAWINGS

FIG. 1 depicts the molecular structure of (a) phosphatidylcholine (b) sodium deoxycholate and (c) benzyl alcohol.
FIG. 2 depicts the effects of a phosphatidylcholine bile salt formulation (PBF) (5.0% highly purified soy derived PC, 4.75% sodium deoxycholate, and 0.9% benzyl alcohol, in sterile water) (not according to the invention) and sodium deoxycholate alone on cultured cell viability: (a) MTS assay measuring viability of keratinocytes exposed to the PBF and sodium deoxycholate alone; (b) lactate dehydrogenase (LDH) assay measuring LDH release by cells exposed to the PBF and sodium deoxycholate alone.
FIG. 3 depicts the effects of PBF (not according to the invention) and sodium deoxycholate alone on primary porcine fat tissue: (a) MTS assay producing purple pigment, indicating living cells, in fat specimens treated with the PBS buffer as negative control (- Cont), sodium deoxycholate alone (DC), the PBF, and Triton detergent as positive control (+ Cont); (b) A comparison of fat cell viability between the different treatments.
FIG. 4 depicts calcein fluorescence in fat specimens treated with sodium deoxycholate alone (DC), PBF (not according to the invention), Triton detergent as positive control (+ Cont), and PBS buffer as negative control (- Cont).
FIG. 5 depicts light microscopy of porcine skin biopsies after treatment revealing (a) control lipocytes and (b) lipocytes after PBF injection (H&E, original magnification, x20) (not according to the invention); (c) control lipocytes and (d) lipocytes after injection of sodium deoxycholate alone (H&E, original magnification, x10); (e) control muscle and (f) muscle after injection of phosphatidylcholine alone (H&E, original magnification, x10) (not according to the invention); (g) fat after injection with Empigen detergent (H&E, original magnification, x20) (not according to the invention).
FIG. 6 depicts a lipoma removed from a patient two days after injection with deoxycholate according to the teachings of the present invention: (a) gross pathology and (b) histology (H&E, original magnification, x20).
FIG. 7 depicts effects of sodium deoxycholate only and sodium deoxycholate-1% phosphatidylcholine solutions (not according to the invention) on melanocytes.
FIG. 8 depicts effects of sodium deoxycholate only and sodium deoxycholate -1% phosphatidylcholine solutions (not according to the invention) on adipocytes.
FIG. 9 depicts effects of addition of phosphatidylcholine to 4.75% sodium deoxycholate solutions on viable adipocytes. This addition is not according to the invention.
FIG. 10 depicts inhibition of adipolysis by pre-incubation with human lipoma fat.
FIG. 11 depicts a kit for reducing a subcutaneous fat accumulation.

### DETAILED DESCRIPTION OF THE INVENTION

The present invention addresses problems of localized fat accumulation in animals, such as humans. In particular, the present invention provides compositions for reducing fat deposits. Such compositions consist essentially of between 0.05 and 1% w/w of a bile salt, more preferably a salt of deoxycholic acid, or more preferably sodium deoxycholate. The amount of such detergent(s) in the composition is an effective amount to dissolve or reduce a subdermal fat deposit. Such effective amount will depend, in part, on the location of target site, size of target site, length of treatment, etc.

Pharmacologically active and biologically compatible detergents include, but are not limited to, lipophilic detergents (whether ionic or non-ionic), hydrophilic detergents (whether ionic or non-ionic), ionic detergents, non-ionic detergents, zwitterionic detergents, glycerides, bile acids and bile salts.

Non-limiting examples of lipophilic detergents include, inter alia, alcohols, polyoxyethylene alkylethers, fatty acids, bile acids, glycerol fatty acid esters, acetylated glycerol fatty acid esters, lower alcohol fatty acids esters, polyethylene glycol fatty acid esters, polyethylene glycol glycerol fatty acid esters, polypropylene glycol fatty acid esters, polyoxyethylene glycerides, lactic acid derivatives of mono/diglycerides, propylene glycol diglycerides, sorbitan fatty acid esters, polyoxyethylene sorbitan fatty acid esters, polyoxyethylene-polyoxypropylene block copolymers, transesterified vegetable oils, sterols, sterol derivatives, sugar esters, sugar ethers, sucroglycerides, polyoxyethylene vegetable oils, polyoxyethylene hydrogenated vegetable oils, reaction mixtures of polyols and at least one member of the group consisting of fatty acids, glycerides, vegetable oils, hydrogenated vegetable oils, and sterols, and mixtures thereof.

Non-limiting examples of non-ionic lipophilic detergents include, inter alia, alkylglucosides, alkylmaltosides, alkylthioglucosides, lauryl macrogolglycerides, polyoxyethylene alkyl ethers, polyoxyethylene alkylphenols, polyethylene glycol fatty acids esters, polyethylene glycol glycerol fatty acid esters, polyoxyethylene sorbitan fatty acid esters, polyoxyethylene-polyoxypropylene block copolymers, polyglycerol fatty acid esters, polyoxyethylene glycerides, polyoxyethylene sterols, derivatives, and analogues thereof, polyoxyethylene vegetable oils, polyoxyethylene hydrogenated vegetable oils, reaction mixtures of polyols and at least one member of the group consisting of fatty acids, glycerides, vegetable oils, hydrogenated vegetable oils, and sterols, tocopherol polyethylene glycol succinates, sugar esters, sugar ethers, sucroglycerides, and mixtures thereof.

Non-limiting examples of ionic hydrophilic detergents include, inter alia, alkyl ammonium salts, bile acids and bile salts, analogues, and derivatives thereof, fatty acid derivatives of amino acids, carnitines, oligopeptides, and polypeptides, glyceride derivatives of amino acids, oligopeptides, and polypeptides, acyl lactylates, mono-, diacetylated tartaric acid esters of mono-, diglycerides, succinoylated monoglycerides, citric acid esters of mono-, diglycerides, alginate salts, propylene glycol alginate, lecithins and hydrogenated lecithins, lysolecithin and hydrogenated lysolecithins, lysophospholipids and derivatives thereof, phospholipids and derivatives thereof, salts of alkylsulphates, salts of fatty acids, sodium docusate, and mixtures thereof.

Non-limiting examples of ionic detergents include, but not limited to, cholate, sodium deoxycholate, sodium dodecylsulfate and C-16 TAB. In a preferred embodiment, a non-limiting example of an ionic detergent useful in an embodiment of the present invention is sodium deoxycholate.

Non-limiting examples of non-ionic detergents include, but not limited to, Brij 35, n-alkyl PEO monoether such as, polyoxylethylen(20)cetyl ether, Lubrol PX, Lubrol WX, nonidet P-40, n-alkyl phenyl PEO such as, octylphenolpoly(ethyleneglycolether)n10, and octylphenolpoly(ethyleneglycolether)n7, tetramethylbutylphenyl PEO, n-octylglucoside, octyl-thioglucopyranoside, tween-80 and tween-20, and alkylaryl polyether alcohol (Triton X-100).

Non-limiting examples of zwitterionic detergents include, but not limited to, 3-[(3-cholamidopropyl)dimethylammonio]propane-sulfonate (CHAPS), N-tetradecyl-N,N-dimethyl-3-ammonio-1-propanesulfonate, cholic acid sulfobetaine, lauryldimethylbetaine (Empigen BB) and zwittergent 3-14.

Non-limiting examples of glycerides include, inter alia, mono-, di- or tri-glycerides. Such triglycerides include, inter alia, vegetable oils, fish oils, animal fats, hydrogenated vegetable oils, partially hydrogenated vegetable oils, synthetic triglycerides, modified triglycerides, fractionated triglycerides, and mixtures thereof.

Non-limiting examples of bile acids include ursodeoxycholic acids, cholic acid, glycolic acid, alcoholic acid, taurocholic acid, deoxycholic acid, glycodeoxycholic acid, taurodeoxycholic acid, chenodeoxycholic acid, glycochenodeoxychloic acid, and taurochenodeoxycholic acid, ursocholic acid, 7-oxolithocholic acid, lithocholic acid 3-sulfate, norcholic acid, bisnorcholic acid, hyocholic acid, and hyodeoxycholic acid.

The compositions disclosed herein preferably consist essentially of pharmaceutically acceptable salts of the detergents. Such salts are meant to be those salts which are within the scope of sound medical judgment, suitable for use in contact with the tissues of humans and animals without undue toxicity, irritation, allergic response, and the like, commensurate with a reasonable benefit/risk ratio, and effective for their intended use. Preferably a composition herein consists essentially of a bile salt, or more preferably deoxycholate salt, or more preferably sodium deoxycholate.

Among detergents, bile salts are preferred as they are particularly potent solubilizers of lipid bilayer membranes. All biologic cell membranes are composed of the same bilipid structure, and are therefore subject to solubilization by detergents. Solubilization of cell membranes by a detergent involves distribution of the detergent between lipid bilayers, destabilization of the bilayer, disintegration, and subsequent formation of mixed micelles (composed of detergent and cell membrane lipid). Bile salts, and other detergents, decrease surface tension at the border of immiscible materials and allow the breakdown of large aggregates into smaller particles. In tissue, these agents dissolve cell membranes and cause cell lysis. An inflammatory response is generated, causing the body to remove the detergent solubilized material.

Bile salts have been used to improve the aqueous solubility of phosphatidylcholine (PC) and more recently, medications like amphotericin B, Taxol, and diazepam. Highly purified phosphatidylcholine can be combined with the secondary bile salt sodium deoxycholate, an anti-microbial, benzyl alcohol, and water to form a stable, mixed micelle preparation that can be rapidly sterilized and used for intravenous administration. Pharmaceutical preparations of this mixture, known as Essentiale and Lipostabil, are marketed in other countries for treatment of liver disease and hyperlipidemia, respectively.

Bile salts may be formed from a bile acid in combination with cations such as inorganic bases, ammonia, organic bases, basic amino acids or the like. Examples of the inorganic bases include alkali metal (*e.g*., Li⁺, Na⁺ and K⁺) and alkaline earth metal (*e.g*., Mg²⁺, Ca²⁺, Sr²⁺, Ba²⁺). Examples of the organic base include procaine, 2-phenylethylbenzylamine, dibenzylethylenediamine, ethanolamine, diethanolamine, tris(hydroxymethyl)aminomethane, polyhydroxyalkylamine, and N-methyl glucosamine. Examples of the basic amino acid include lysine, arginine, ornithine and histidine. Example of other salts includes halogen ions. Non limiting examples of the salts that can be combined with bile acids to form bile salts include, but are not limited to, ammonium chloride, ammonium sulphate, sodium chloride, sodium bromide, sodium iodide, sodium fluoride, sodium citrate, sodium sulphate, sodium carbonate, sodium bicarbonate, sodium acetate, sodium nitrate, sodium nitrite, potassium acetate, potassium carbonate, potassium dichromate, potassium chloride, potassium bromide, magnesium bromide, magnesium chloride, potassium iodide, sodium fluoride, hydroxyl amine hydrochloride, sodium fluoride, sodium silicate, diammonium phosphate, sodium thiocyanate, potassium thiocyanate, lithium thiocyanate, sodium borohydride, calcium carbonate, barium carbonate, sodium dihydrogen phosphate, lithium chloride, lithium bromide, and lithium iodide. In some embodiments, the salt ion that combines with the bile acid to form a bile salt is a cation. Non limiting examples of cations include sodium (Na⁺), potassium (K⁺), lithium (Li⁺), magnesium (Mg²⁺), calcium (Ca²⁺), barium (Ba²⁺), strontium (Sr²⁺), and ammonium (NH₄⁺).

Examples of bile salts that can be formed from a combination of a bile acid and a cation, include, but are not limited to, sodium cholate, sodium deoxycholic, sodium cholic, sodium chenodeoxycholic, sodium 7-alpha-dehydroxylate chenodeoxycholic, sodium lithocholic, sodium ursodeoxycholic, potassium deoxycholate, potassium cholate, potassium chenodeoxycholate, potassium 7-alpha-dehydroxylate chenodeoxycholate, potassium lithocholate, potassium ursodeoxycholate, lithium deoxycholate, lithium cholate, lithium chenodeoxycholate, lithium 7-alpha-dehydroxylate chenodeoxycholate, lithium lithocholate, lithium ursodeoxycholate, magnesium deoxycholate, magnesium cholate, magnesium chenodeoxycholate, magnesium 7-alpha-dehydroxylate chenodeoxycholate, magnesium lithocholate, magnesium ursodeoxycholate, ammonium cholate, ammonium deoxycholate, ammonium cholate, ammonium chenodeoxycholate, ammonium 7-alpha-dehydroxylate chenodeoxycholate, ammonium lithocholate, ammonium ursodeoxycholate, dihydroxy- and trihydroxy- and taurine or glycine conjugates of any of the above. Preferably a bile salt of the invention is sodium deoxycholate. Any of the above bile salts can be used in the compositions herein.

Other examples of bile salts include steroids having 1-3 hydroxyl groups and a five carbon atom side chain terminating in a carboxyl group, which can be conjugated to glycine or taurine.

Also disclosed are esters of a bile acid. Such esters include, but are not limited to, esters formed by a combination of a bile acid with a hydrogen of a COOH group optionally substituted C₁-C₆ alkyl, C₂-C₆ alkenyl, C₃-C₁₀ cycloalkyl, C₃-C₁₀ cycloalkyl(C₁-C₆)alkyl, optionally substituted C₆-C₁₀ aryl, optionally substituted C₇-C₁₂ aralkyl, di(C₆-C₁₀)arylmethyl, tri(C₆-C₁₀)arylmethyl, or a substituted silyl.

Examples of the optionally substituted C₁₋₆ alkyl include *e.g.*, methyl, ethyl, n-propyl, n-butyl, t-butyl, n-pentyl, and n-hexyl, each maybe substituted with benzyloxy, C₁₋₄ alkylsulfonyl (*e.g*., methanesulfonyl), trimethylsilyl, halogen (*e.g*., F, Cl, and Br), acetyl, nitrobenzoyl, mesylbenzoyl, phthalimide, succinoylimide, benzenesulfonyl, phenylthio, di-C₁₋₄ alkylamino (*e.g*., dimethylamino), pyridyl, C₁₋₄ alkylsulfinyl (*e.g*., methanesulfinyl), cyano and the like. Such substituted C₁₋₆ alkyl include *e.g.*, benzyloxymethyl, 2-methanesulfonylethyl, 2-trimethylsilylethyl, 2,2,2-trichloroethyl, 2-iodoethyl, acetylmethyl, p-nitrobenzoylmethyl, p-mesylbenzoylmethyl, phthalimidemethyl, succinoylimidemethyl, benzenesulfonylmethyl, phenylthiomethyl, and 1-dimethylaminoethyl. The above C₂₋₆ alkenyl includes *e.g.*, vinyl, aryl, 1-propenyl, isopropenyl, 1-butenyl, 2-butenyl, 1,1-dimethylaryl, 3-methyl and 3-butenyl. The above C₃₋₁₀ cycloalkyl includes *e.g.*, cyclopropyl, cyclobutyl, cyclopentyl, cyclohexyl, cycloheptyl, norbornyl, and adamantyl. The above C₃₋₁₀ cycloalkyl(C₁₋₆)alkyl includes *e.g*., cyclopropylmethyl, cyclopentylmethyl, and cyclohexylmethyl. The above C₆₋₁₀ aryl includes *e.g.*, phenyl, -naphthyl, 8-naphthyl, and biphenyl, each may be substituted with nitro, halogen (*e.g*., F, Cl, and Br) or the like, and such substituted aryl includes *e.g.*, p-nitrophenyl and p-chlorophenyl. The above optionally substituted C₇₋₁₂ aralkyl includes *e.g.*, benzyl, 1-phenylethyl, 2-phenylethyl, phenylpropyl and naphthylmethyl, each maybe substituted with nitro, C₁₋₄ alkoxy (*e.g*., methoxy), C₁₋₄ alkyl (*e.g*., methyl, ethyl), hydroxy or the like. Such substituted group is exemplified by p-nitrobenzyl, p-methoxybenzyl (PMB), or 3,5-di-t-butyl-4-hydroxybenzyl. The above di(C₆₋₁₀ aryl)methyl includes benzhydryl and the C₆₋₁₀ arylmethyl includes trityl, and the substituted silyl includes trimethylsilyl and tert-butyldimethylsilyl. Examples of the active ester include organic phosphate esters (*e.g*., diethoxy phosphate ester and diphenoxy phosphate ester), cyanomethyl ester, and the active thioester includes esters formed with aromatic heterocyclicthio compound (*e.g.,* 2-pyridilthio ester).

Derivatives of bile acids can also be used as detergents. Such derivatives include bile acid halides, bile acid azides, bile acid anhydrides, mixed bile acid anhydride, bile acid amide, and bile acid thioester. The bile acid halide includes bile acid chloride such as deoxycholic chloride and bile acid bromide such as deoxycholic acid bromide; the mixed bile acid anhydride includes mixed monoalkylcarboxylic acid anhydride, mixed alphatic carboxylic acid anhydride, aromatic carboxylic acid anhydride, organic sulfonic acid anhydride, and wherein the active amide includes amide formed with heterocyclic compound containing N atom.

The pharmacologically active detergents (including bile salts) are preferably micelle-forming compounds. Micelles can significantly increase the solubility of hydrophobic molecules not ordinarily soluble in water (*e.g*., the lipids that comprise cell membranes) by burying their hydrophobic portions away from aqueous solvent (*e.g*., water). In some embodiments, a composition herein comprises homogenous micelles (micelles produced by a single detergent). In some embodiments, a composition herein comprises mixed micellar formations (micelles produced by two or more compounds - one of which is a detergent).

In some embodiments, a composition comprising micelles with an average size in the range of 10⁻⁹ m-10⁻⁵m, 5 x 10⁻⁹ m - 10⁻⁶ m, 10 x 10⁻⁹ m - 10⁻⁷ m, or 50 x 10⁻⁹ m - 10 x 10⁻⁸ m. In some embodiments, an average size of a micelle in a composition of the present invention may be up to 10⁻⁵, 10⁻⁶, 10⁻⁷, 10⁻⁸, 10⁻⁹ m. In some embodiments, an average size of a micelle in a composition of the present invention may be greater than 10⁻⁵, 10⁻⁶, 10⁻⁷, 10⁻⁸, 10⁻⁹ m. Moreover, the shape of the micelle can vary and can be, for example, prolate, oblate or spherical; spherical micelles are most typical.

Table 1 below illustrates several detergents, their monomeric molecular weight as monomers, and their critical micellar concentration (CMCs), which is the minimum concentration at which the detergent is predominantly in the form of micelles.

**TABLE 1**

| **Detergent Name** | **Molecular Weight (AMU)** | **Micellar Molecular Weight (AMU)** | **CMC in H2O (M)** |
|---|---|---|---|
| **Anionic** | | | |
| Cholate | 430 | 4300 | 1.4x10-2 |
| Deoxycholate | 415-432 | 4200 | 5x10-3 |
| Sodium dodecvl sulfate | 288 | 18000 | 8.3x10-3 |

| Cationic | | | |
|---|---|---|---|
| C16-TAB | 365 | 62000 | 1x10-3 |

| Amphoteric (Zwitterionic) | | | |
|---|---|---|---|
| Cholic acid-sulfobetaine | 615 | 6150 | 4x10-3 |
| Cholic acid-sulfobetaine | 631 | 6940 | 8x10-3 |
| Lysophophatidylcholine | 495 | 92000 | 7x10-6 |
| Zwitergent 3-14 | 364 | 30000 | 3x10-4 |

| Non-Ionic | | | |
|---|---|---|---|
| Brij 35 | 1225 | 49000 | 9x10-5 |
| polyoxylethylen(20)cetyl ether | 1120 | 82000 | 7.7x10-5 |
| Lubrol PX | 582 | 64000 | 1x10-4 |
| Nonidet P-40 | 603 | 90000 | 3x10-4 |
| Octylphenolpoly(ethyleneglycolether)n10 | 647 | 90000 | 0.2x10-3 |
| Octylphenolpoly(ethyleneglycolether)n7 | 515 | | 0.2x10-3 |
| n-Octylglucoside | 292 | 8000 | 14.5x10-3 |
| Octyl-thioglucopyranoside | 308 | | 9x10-3 |
| Tween-80 | 1310 | 76000 | 1.2x10-5 |
| Tween-20 | 1228 | | 6.0x10-5 |

In some embodiments, the concentration of the one or more pharmacologically active detergents (*e.g*., bile salts) in a composition is such that it is at approximately the CMC concentration (i.e., +/- 5 mM), or above the CMC level (*e.g*., more than 1, 5, 10 15, 20, 25, 30, 35, 40, 50, 55, 60, 65, 70, 75, 80, 85, 90, 99, 150, 200, 400, 800, 1600, 3200, 6800, 13,600, 27,200, or 54,400%, above the CMC concentration level).

In some embodiments, at least 10, 15, 20, 25, 30, 35, 40, 45, 50, 55, 60, 65, 70, 75, 80, 85, 90, 95, or 99% of the detergents (*e.g*., bile salts), in the compositions are in micellar formation. In other embodiments, up to 90, 80, 70, 60, 50, 40, 30, 20, 10, or 5% of the detergents (*e.g*., bile salt(s)), in the compositions are in micellar formation. In other embodiments, about 10-90, 20-80, 30-70, 40-60, or about 50% of the detergents (*e.g*., bile salts), of the compositions are in micellar formation.

In some embodiments, an average molecular weight of a micelle in a composition of the present invention maybe up to 100,000, 50,000, 40,000, 30,000, 20,000, 10,000, 9,000, 8,000, 7,000, 6,000, 5,000, 4,000, 3,000, 2,000, 1,000, or 500 Daltons (D). In some embodiments, an average molecular weight of a micelle in a composition of the present invention may be greater than 500, 1,000, 1,500, 2,000, 2,500, 3,000, 3,500, 4,000, 4,500, 5,000, 5,500, 6,000, 6,500, 7,000, 7,500, 8,000, 8,500, 9,000, 9,500, 10,000, or 15,000 D. In some embodiments, an average molecular weight of a micelle in a composition of the present invention may be in the range of 100-20,000, 1,000-10,000, 2,000-1,000, or 3,000-5,000 D.

Preferably, the concentration of the one or more bile salts in a composition of the invention is in the range from 0.05-1% or 0.5-1% w/w. It is understood that the final concentration is dependent on many factors known to persons skilled in the art including, but not limited to, location and size of the target site.

In some embodiments, the compositions herein do not include phosphatidylcholine, phospholipids, or lipids. In some embodiments, the compositions herein do not include lipase or colipase, lipase and colipase, a platelet activating factor inhibitor, or all enzymes. In some embodiments, the compositions herein do not include testosterone, prostaglandins, progesterone, estrogen, or steroids. In some embodiments, the compositions herein do not include tyloxapol or another alkylaryl polyether alcohol. In some embodiments, the compositions herein do not include carprofen, a non-steroidal anti-inflammatory compound, or any anti-inflammatory compound. In some embodiments, the compositions herein do not include lipids, oils, fatty acids, triglycerides or polyacrylamides or a combination thereof. Any of the above or all of the above may be excluded from the composition herein.

The compositions herein can be formulated for various types of delivery *e.g.*, topical, subcutaneous, subdermal, intra-adipocyte, intramuscular etc. by any means known in the art. Such formulations can be in the form of a tablet, powder, gel, solution, cream, vapor, ointment, etc. Preferably, the bile salt, more preferably a salt of deoxycholic acid, or more preferably sodium deoxycholate, are formulated into a solution. Preferably, such solution is aqueous. The term "aqueous" as used herein refers to a solution which is a homogenous mixture prepared by dissolving a solid or a liquid in water such that the molecules of the solute or dissolved substance are dispersed among those of water. Pharmacologically acceptable aqueous vehicles for the compositions of the present invention can include, for example, any liquid solution that is capable of dissolving the bile salt and is not toxic to the particular individual receiving the formulation. Examples of pharmaceutically acceptable aqueous vehicles include, without limitation, saline, water, benzyl alcohol and acetic acid. Typically, pharmaceutically acceptable aqueous vehicles are sterile.

For delivery into humans (*e.g*., transdermally or subcutaneously), the compositions herein are formulated with one or more pharmaceutical excipients.

Examples of pharmaceutical excipients include: buffers, diluents, lubricating agents, solubilizers, solvents; surfactants, penetration enhancers, polymers, dispersion agents, wetting agents, emulsifying and suspending agents, and preserving agents. Examples of dispersion agents include, but are not limited to, hyaluronidase and collagenase. Hyaluronidase functions to augment tissue permeability and spread or dispersion of other drugs. Collagenase has been used to isolate adipocytes from subcutaneous fat and does not have lytic effects on adipocytes themselves. Additionally hyaluronidase and collagenase can facilitate healing by accelerating reduction of necrotic tissue after treatment with the detergent formulations of the present invention. In some embodiments, the dispersion agents, such as collagenase, are administered prior to the administration of the detergent(s) herein. This may help release the adipocytes from the extracellular matrix to enhance their exposure to the detergent(s).

Examples of wetting agents include, but not limited to, acetylene glycols, acetylene alcohols, glycol ethers, alkylene glycols, lower alcohols, and nonionic surface active agents. Further, other alcohols, water-soluble organic solvents, anionic surface active agents, cationic surface active agents, amphoteric surface active agents, and saccharides may be used singly or in combination of two or more thereof. Some examples of lower alcohols include but not limited to, methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol and water-soluble organic solvents such as ethylene glycol, diethylene glycol, triethylene glycol, propylene glycol, tetraethylene glycol, polyethylene glycol, dipropylene glycol, polypropylene glycol, hexylene glycol, thiodiglycol, glycerin, and 1,2,6-hexanetriol. In preferred embodiments, an excipient is an organic solvent, more preferably organic alcohols, or more preferably benzyl alcohol.

Examples of lubricating agents include, but not limited to, talc, magnesium stearate, stearic acid, and silica gel.

Examples of the preserving agents include, but not limited to, benzalkonium chloride, benzethonium chloride, chlorobutanol, phenylethyl alcohol, paraoxybenzoic acid esters, and the like. Few examples of penetration enhancers are, but not limited to, dimethylsulfoxide, n-decyl methyl sulfoxide, N,N-dimethylacetamide, N,N-methyl-2-pyrrolidone, and octylphenylpolyethylene glycols.

Examples of suitable emulsifiers include, but not limited to, sodium lauryl sulfate, sodium cetyl stearyl sulfate, sucrose stearate, and Polysorbate 80.

Examples of anionic surfactants include, but are not limited to, fatty acid soaps, N-acylamino acids, alkyl ether carboxylates, acylated peptides, alkylbenzenesulfonates, alkylnaphthalenesulfonates, naphthalenesulfonate-formalin polymerization condensates, melaminesulfonate-formalin polymerization condensates, dialkylsulfosuccinic ester salts, alkylsulfoacetates, α-olefinsulfonates, N-acylmethyltaurine, sulfated oil, higher alcohol sulfuric ester salts, secondary higher alcohol sulfuric ester salts, alkylether sulfates, secondary higher alcohol ethoxysulfates, polyoxyethylene alkylphenyl ether sulfates, monoglysulfates, fatty acid alkylolamide sulfuric ester salts, alkyl ether phosphoric ester salts, and alkylphosphoric ester salts.

Examples of nonionic surfactants include, but are not limited to, polyoxyethylene alkyl ethers, single chain length polyoxyethylene alkyl ethers, polyoxyethylene secondary alcohol ethers, polyoxyethylene alkylphenyl ethers, polyoxyethylene sterol ethers, polyoxyethylene lanoline derivatives, ethylene oxide derivatives of alkylphenol-formalin condensates, polyoxyethylene polyoxypropylene block polymers, polyoxyethylene polyoxypropylene alkyl ethers, polyoxyethylene glycerol fatty acid esters, polyoxyethylene sorbitan fatty acid esters, polyoxyethylene sorbitol fatty acid esters, polyethyleneglycol fatty acid esters, fatty acid monoglycerides, polyglycerol fatty acid esters, sorbitan fatty acid esters, propyleneglycol fatty acid esters, sucrose fatty acid esters, fatty acid alkanolamides, polyoxyethylene fatty acid amides, polyoxyethylene alkylamines, alkylamine oxides, polyoxyethylene castor oil derivatives, polyvinylpyrrolidone, polyvinyl alcohol, carboxymethyl cellulose, lecithin, gelatin, and hyaluronic acid. Any and all of the above may be used in combination with one another as appropriate.

Other examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, phosphatidylcholine, cellulose, sterile water, syrup, and methyl cellulose.

The pharmacologically active bile salt, more preferably a salt of deoxycholic acid, or more preferably sodium deoxycholate is administered at various concentrations such that a therapeutically effective amount is delivered to a subject. A therapeutically effective amount is the amount of detergent(s) effective to reduce the size of a subcutaneous fat deposit.

When the composition is formulated as a solution (preferably aqueous solution), it may be in a container, such as a syringe or a syringe loadable container. A solution in a container or a unit dose of a solution herein is preferably up to 500, 250, 100, 25, 10, or 2.5 mL.

Also disclosed are compositions/solutions which can also include additional active ingredient(s) or second therapeutic agent(s). Such second therapeutic agents are selected from the group consisting of: anti-microbial agents, vasoconstrictors, anti-thrombotic agents, anti-coagulation agents, suds-depressants, anti-inflammatory agents, analgesics, dispersion agents, anti-dispersion agents, penetration enhancers, steroids, tranquilizers, muscle relaxants, and anti-diarrhea agents.

### Additional Active Ingredients

In some embodiments of the present invention, a composition can be co-administered, and/or co-marketed with one or more additional active ingredients, such as, for example, anti-microbial agents, vasoconstrictors, anti-thrombotic agents, anti-coagulation agents, suds-depressants, anti-inflammatory agents, analgesics, dispersion agents, anti-dispersion agents, penetration enhancers, steroids, tranquilizers, muscle relaxants, and anti-diarrhea agents.

Examples of anti-microbial agents include, but not limited to, anti-bactericidal agents, anti-fungal agents, anti-viral agents and the like, and are preferably efficacious against a broad spectrum of microbes.

Examples of anti-fungal agents include dithiocarbamates, phthalimides, dicarboximides, organophosphates, benzimidazoles, carboxanilides, phenylamides, phosphites, and the like.

Examples of anti-bacterial agents include, but are not limited to, erythromycin, clarithromycin, penicillins, cephalosporins, aminoglycosides, sulfonamides, macrolides, tetracyclins, lincosides, quinolones, chloramphenicol, vancomycin, metronidazole, rifampin, isoniazid, spectinomycin, trimethoprim, sulfamethoxazole, penems, carbapenems, monobactams mupirocin, neomycin sulfate bacitracin, polymyxin B, 1-ofloxacin, tetracyclines (chlortetracycline hydrochloride, oxytetracycline hydrochloride and tetrachcycline hydrochoride), clindamycin phosphate, gentamicin sulfate, benzalkonium chloride, benzethonium chloride, hexylresorcinol, methylbenzethonium chloride, phenol, quaternary ammonium compounds, triclocarbon, triclosan, tea tree oil, and their pharmaceutically acceptable salts, and pharmaceutically acceptable esters thereof.

Other examples of anti-bacterial agents include, but are not limited to, Acrofloxacin, Amoxicillin plus clavulanic acid (i.e., Augmentin), Amikacin, Ampicillin, Apalcillin, Apramycin, Astromicin, Arbekacin, Aspoxicillin, Azidozillin, Azithromycin, Azlocillin, Bacitracin, Benzathine penicillin, Benzylpenicillin, Carbencillin, Cefaclor, Cefadroxil, Cefalexin, Cefamandole, Cefaparin, Cefatrizine, Cefazolin, Cefbuperazone, Cefcapene, Cefdinir, Cefditoren, Cefepime, Cefetamet, Cefixime, Cefmetazole, Cefminox, Cefoperazone, Ceforanide, Cefotaxime, Cefotetan, Cefotiam, Cefoxitin, Cefpimizole, Cefpiramide, Cefpodoxime, Cefprozil, Cefradine, Cefroxadine, Cefsulodin, Ceftazidime, Ceftriaxone, Cefuroxime, Chlorampenicol, Chlortetracycline, Ciclacillin, Cinoxacin, Ciprofloxacin, Clarithromycin, Clemizole penicillin, Clindamycin, Cloxacillin, Daptomycin, Demeclocycline, Desquinolone, Dibekacin, Dicloxacillin, Dirithromycin, Doxycycline, Enoxacin, Epicillin, Erthromycin, Ethambutol, Fleroxacin, Flomoxef, Flucloxacillin, Flumequine, Flurithromycin, Fosfomycin, Fosmidomycin, Fusidic acid, Gatifloxacin, Gemifloxaxin, Gentamicin, Imipenem, Imipenem plus Cilistatin combination, Isepamicin, Isoniazid, Josamycin, Kanamycin, Kasugamycin, Kitasamycin, Latamoxef, Levofloxacin, Lincomycin, Linezolid, Lomefloxacin, Loracarbaf, Lymecycline, Mecillinam, Meropenem, Methacycline, Methicillin, Metronidazole, Mezlocillin, Midecamycin, Minocycline, Miokamycin, Moxifloxacin, Nafcillin, Nafcillin, Nalidixic acid, Neomycin, Netilmicin, Norfloxacin, Novobiocin, Oflaxacin, Oleandomycin, Oxacillin, Oxolinic acid, Oxytetracycline, Paromycin, Pazufloxacin, Pefloxacin, Penicillin G, Penicillin V, Phenethicillin, Phenoxymethyl penicillin, Pipemidic acid, Piperacillin, Piperacillin and Tazobactam combination, Piromidic acid, Procaine penicillin, Propicillin, Pyrimethamine, Rifabutin, Rifamide, Rifampicin, Rifamycin SV, Rifapentene, Rokitamycin, Rolitetracycline, Roxithromycin, Rufloxacin, Sitafloxacin, Sparfloxacin, Spectinomycin, Spiramycin, Sulfadiazine, Sulfadoxine, Sulfamethoxazole, Sisomicin, Streptomycin, Sulfamethoxazole, Sulfisoxazole, Synercid (Quinupristan-Dalfopristan combination), Teicoplanin, Telithromycin, Temocillin, Tetracycline, Tetroxoprim, Thiamphenicol, Ticarcillin, Tigecycline, Tobramycin, Tosufloxacin, Trimethoprim, Trimetrexate, Trovafloxacin, Vancomycin, and Verdamicin.

Examples of vasoconstrictor agents include dihydroergotamine, ergotamine and methysergide, and pharmaceutically-acceptable salts thereof.

Examples of anti-thrombotic agents include argatroban, iloprost, lamifiban, taprostene, tirofiban, tissue plasminogen activator (natural or recombinant), tenecteplase (TNK), and lanoteplase (nPA), factor VIIa inhibitors, factor Xa inhibitors, thrombin inhibitors (such as hirudin and argatroban), PAI-1 inhibitors (i.e., inactivators of tissue plasminogen activator inhibitors), alpha2-antiplasmin inhibitors, streptokinase, urokinase and prourokinase, and anisoylated plasminogen streptokinase activator complex, anti-coagulants (*e.g*., hirudin, heparin, etc.), plasminogen activators (*e.g*., t-PA, urokinase, etc.), fibrinolytic enzymes (*e.g*., plasmin, subtilisin, etc.), anti-platelet-aggregation agents (*e.g*., prostacyclin, aspirin, etc.) and the like.

Examples of anti-coagulation agents include cilostazol, clopidogrel, ticlopidine, tirofiban, eptifibatide, abciximab, anagrelide, dipyridamole, aspirin, dipyridamole/aspirin, dalteparin, enoxaparin, tinzaparin, heparin (various), danaparoid, antithrombin III, lepirudin, argatroban, bivalirudin, warfarin, anisidione, alteplase, reteplase, tenecteplase, drotrecogin, anistreplase, streptokinase, urokinase, and combinations thereof.

Examples of suds-depressants include monocarboxylic fatty acid and soluble salts thereof. The monocarboxylic fatty acids and their salts can have hydrocarbyl chains of about 1 to about 50 carbon atoms, about 10 to about 24 carbon atoms, or about 12 to about 18 carbon atoms. Suitable salts include the alkali metal salts such as sodium, potassium, and lithium salts, and ammonium and alkanolammonium salts. Additional suds-depressants include, for example, high molecular weight hydrocarbons such as paraffin, fatty acid esters (*e.g*., fatty acid triglycerides), fatty acid esters of monovalent alcohols, aliphatic C₁₈-C₄₀ ketones (*e.g*., stearone), etc. Other suds-depressants include N-alkylated amino triazines such as tri- to hexa-alkylmelamines or di- to tetra-alkyldiamine chlortriazines formed as products of cyanuric chloride with 1-5 or 2-3 moles of a primary or secondary amine containing 1 to 24 carbon atoms, propylene oxide, and monostearyl phosphates such as monostearyl alcohol phosphate ester and monostearyl di-alkali metal (*e.g*., K⁺, Na⁺, and Li⁺) phosphates and phosphate esters. The hydrocarbons such as paraffin (including mixtures of true paraffins and cyclic hydrocarbons) and haloparaffin can be utilized in liquid form. It is also known to utilize waxy hydrocarbons, preferably having a melting point below about 100° C. The hydrocarbons constitute a preferred category of suds-suppressor for detergent compositions. The hydrocarbons can include aliphatic, alicyclic, aromatic, and heterocyclic saturated or unsaturated hydrocarbons having from about 12 to about 70 carbon atoms. Another example of suds suppressors comprises silicone suds suppressors. This category includes the use of polyorganosiloxane oils, such as polydimethylsiloxane, dispersions or emulsions of polyorganosiloxane oils or resins, and combinations of polyorganosiloxane with silica particles wherein the polyorganosiloxane is chemisorbed or fused onto the silica. Examples also include, but are not limited to, silicones, and silica-silicone mixtures. Silicones can be generally represented by alkylated polysiloxane materials while silica is normally used in finely divided forms exemplified by silica aerogels and xerogels and hydrophobic silicas of various types. Silicone suds controlling agent, DC-544, is commercially available from Dow Corning, which is a siloxane-glycol copolymer. Other suds suppressors include mixtures of silicone oils and 2-alkyl-alcanols. Suitable 2-alkyl-alkanols are 2-butyl-octanol which are commercially available.

Examples of anti-dispersion agents include, but are not limited to, sucrose, glyercerol, and glycerin.

Examples of steroids include, but are not limited to, betamethasone, chloroprednisone, clocortolone, cortisone, desonide, dexamethasone, desoximetasone, difluprednate, estradiol, fludrocortisone, flumethasone, flunisolide, fluocortolone, fluprednisolone, hydrocortisone, meprednisone, methylprednisolone, paramethasone, prednisolone, prednisone, pregnan-3-alpha-ol-20-one, testosterone, and triamcinolone, estradiol, estron, estriol, polyestradiol, polyestriol, dienestrol, diethylstilbestrol, dihydroergosterone, cyproterone, danazol, testosterone, progesterone, norethindrone, levonorgestrol, ethynodiol, norgestimate, gestanin, 3-keton-desogestrel, demegestone, promethoestrol, testosterone, spironolactone, and esters thereof, budesonide, rofleponide, rofleponide palmitate, ciclesonide, momethasone furoate, fluticasone propionate, tipredane, fluocinolone acetonide, flunisolide, flumethasone, dexamethasone, beclomethasone dipropionate, deflazacort, cortivazol, or cortisol and/or hydrocortisol, prednisone, fluorometholone acetate, dexamethasone sodium phosphate, suprofen, fluorometholone, and medrysone, optionally in their pure isomeric forms (where such forms exist) and pharmaceutically acceptable salts thereof.

Examples of anti-inflammatory agents include both steroidal and non-steroidal anti-inflammatory agents. Suitable steroidal anti-inflammatory agent include, but are not limited to, corticosteroids such as hydrocortisone, hydroxyltriamcinolone alphamethyl dexamethasone, dexamethasone-phosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclarolone acetonide, fludrocortisone, flumethasone pivalate, fluosinolone acetonide, fluocinonide, flucortine butylester, fluocortolone, fluprednidene (fluprednylidene)acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenalone acetonide, medrysone, amciafel, amcinafide, betamethasone and the balance of its esters, chlorprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylproprionate, hydrocortamate, meprednisone, paramethasone, prednisolone, prednisone, beclomethasone dipropionate, betamethasone dipropionate, triamcinolone, and mixtures thereof.

Suitable non-steroidal anti-inflammatory agents include, but are not limited to, oxicams, such as piroxicam, isoxicam, tonexicam, sudoxicam, and CP-14,3 04; salicylates, such as salicylic acid, aspirin, disalcid, benorylate, trilisate, safapryn, solprin, diflunisal, and fendosal; acetic acid derivatives, such as diclofenac, fenclofenac, indomethacin, sulindac, tolmetin, isoxepac, furofenac, tiopinac, zidometacin, acematacin, fentiazac, zomepiract, clidanac, oxepinac, and felbinac; the fenamates, such as mefenamic, meclofenamic, flufenamic, niflumic, and tolfenamic acids; propionic acid derivates, such as ibuprofen, naproxen, benoxaprofen, flurbiprofen, ketoprofen, fenoprofen, fenbufen, indoprofen, pirprofen, carprofen, oxaprozin, pranoprofen, miroprofen, tioxaprofen, suprofen, alminoprofen, and tiaprofenic; and pyrazoles, such as phenybutazone, oxyphenbutazone, feprazone, azapropazone, and trimethazone. Mixtures of these nonsteroidal anti-inflammatory agents can also be employed, as well as the pharmaceutically-acceptable salts and esters thereof.

Examples of analgesics to reduce discomfort due to inflammation include, but are not limited to, lidocaine, mepivacaine, bupivacaine, procaine, chloroprocaine, etidocaine, prilocaine dyclonine, hexylcaine, procaine, cocaine, ketamine, morphine, pramoxine, propophol, phenol, naloxone, meperidine, butorphanol or pentazocine, or morphine-6-glucuronide, codeine, dihydrocodeine, diamorphine, dextropropoxyphene, pethidine, fentanyl, alfentanil, alphaprodine, buprenorphine, dextromoramide, diphenoxylate, dipipanone, heroin (diacetylmorphine), hydrocodone (dihydrocodeinone), hydromorphone (dihydromorphinone), levorphanol, meptazinol, methadone, metopon (methyldihydromorphinone), nalbuphine, oxycodone (dihydrohydroxycodeinone), oxymorphone (dihydrohydroxymorphinone), phenadoxone, phenazocine, remifentanil, tramadol, tetracaine, and mixtures thereof, as well as pharmaceutically acceptable salts and esters thereof. A composition can include an analgesic selected from the group consisting of lidocaine, hydromorphone, oxycodone, morphine and pharmaceutically-acceptable salts thereof.

Examples of tranquilizer and sedative drugs include, but are not limited to, chlordiazepoxide, benactyzine, benzquinamide, flurazepam, hydroxyzine, loxapine, promazine, and acceptable salts and esters thereof.

Examples of muscle relaxants include, but are not limited to, cinnamedrine, cyclobenzaprine, flavoxate, orphenadrine, papaverine, mebeverine, idaverine, ritodrine, dephenoxylate, dantrolene, azumolene, and pharmaceutically-acceptable salts thereof.

Examples of anti-diarrhea agents include, but are not limited to, loperamide and pharmaceutically-acceptable salts thereof.

The examples herein and other active agents can be co-formulated or co-administered with the one or more pharmacologically active detergents (*e.g* bile acids or bile salts). When co-formulated with a detergent (*e.g*., bile salt) the additional agent can be up to 20, 19, 18, 17, 16, 15,14, 13, 12, 11, 10, 9, 8, 7, 6, 5, 4, 3, 2, 1, 0.5, 0.4, 0.3, 0.2, 0.1, 0.09, 0.08, 0.07, 0.06, 0.05, 0.04, 0.03, 0.02, 0.01, 0.009, 0.008, 0.007, 0.006, 0.005, 0.004, 0.003, 0.002, 0.001, 0.0009, 0.0008, 0.0007, 0.0006, 0.0005, 0.0004, 0.0003, 0.0002, or 0.0001% w/w, w/v or v/v or at greater than 0.0001, 0.0002, 0.0003, 0.0004, 0.0005, 0.0006, 0.0007, 0.0008, 0.0009, 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 1.0, 1.25, 1.50, 1.75, 2.0, 2.25, 2.50, 2.75, 3.0, 3.25, 3.50, 3.75, 4.0, 4.25, 4.50, 4.75, 5.0, 5.25, 5.50, 5.75, 6.0, 6.25, 6.50, 6.75, 7.0, 7.25, 7.50, 7.75, 8.0, 8.25, 8.50, 8.75, 9.0, 9.25, 9.50, 9.75,10, 10.25, 10.50, 10.75, 11, 11.25, 11.50, 11.75, 12, 12.25, 12.50, 12.75, 13, 13.25, 13.50, 13.75, 14, 14.25, 14.50, 14.75, 15, 15.25, 15.50, 15.75, 16, 16.25, 16.50, 16.75, 17, 17.25, 17.50, 17.75, 18, 18.25, 18.50, 18.75, 19, 19.25, 19.50, 19.75, or 20% (w/w, w/v or v/v) of the composition.

The additional active ingredient(s) can be co-formulated with the one or more pharmacologically active detergents (*e.g*., bile acids or bile salts) such that they are at a concentration in the range of from approximately 0.001-50, 0.001-40, 0.01-30, 0.02-29, 0.03-28, 0.04-27, 0.05-26, 0.06-25, 0.07-24, 0.08-23, 0.09-22, 0.1-21, 0.2-20, 0.3-19, 0.4-18, 0.5-17, 0.6-16, 0.7-15, 0.8-14, 0.9-12, or 1-10% w/w, w/v or v/v of the composition.

A composition may contain up to 10, 9.5, 9.0, 8.5, 8.0, 7.5, 7.0, 6.5, 6.0, 5.5, 5.0, 4.5, 4.0, 3.5, 3.0, 2.5, 2.0, 1.5, 1.0, 0.95, 0.9, 0.85, 0.8, 0.75, 0.7, 0.65, 0.6, 0.55, 0.5, 0.45, 0.4, 0.35, 0.3, 0.25, 0.2, 0.15, 0.1, 0.09, 0.08, 0.07, 0.06, 0.05, 0.04, 0.03, 0.02, 0.01, 0.009, 0.008, 0.007, 0.006, 0.005, 0.004, 0.003, 0.002, 0.001, 0.0009, 0.0008, 0.0007, 0.0006, 0.0005, 0.0004, 0.0003, 0.0002, or 0.0001 grams of non-detergent active ingredient(s) or more than 0.0001, 0.0002, 0.0003, 0.0004, 0.0005, 0.0006, 0.0007, 0.0008, 0.0009, 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, or 10 grams of the additional active ingredient(s) or at a range between about 0.0001-10, 0.0005-9, 0.001-8, 0.005-7, 0.01-6, 0.05-5, 0.1-4, 0.5-4, or 1-3 grams of non-detergent active ingredient(s).

### Formulations

The bile salt of the invention is formulated for subcutaneous injection directly into fat deposits. Formulations for injection can be presented in unit dosage form, for example, in ampoules, syringe loadable containers, or in multi-dose containers, with an added preservative. Injectable formulations can take such forms as suspensions, solutions, or emulsions in oily or aqueous vehicles, and can contain agents such as suspending, stabilizing anti-dispersion and/or dispersion agents.

The compositions herein can be formulated so as to provide quick, sustained or delayed release of the active ingredient after administration to the patient by employing procedures known in the art. In some embodiments, a composition herein is formulated for slow release. A slow release formulation or a biodegradable controlled release dosage form provides a composition for prolonging an effect of a fat-solubilizing effective amount of a bile salt *in vivo* at a desired site of treatment. In some embodiments, the detergents herein (*e.g*. bile salts thereof) are formulated with a pharmaceutically acceptable augmenting agent which is effective to further prolong the duration of effect of the detergent(s). The controlled release formulation can be formed into slabs, rods, pellets, microparticles, (*e.g*., microspheres, microcapsules), spheroids, pastes solution, spray, patch, etc. Such formulations can be used to form a suspension in isotonic saline or other physiological buffer or a solution acceptable for subdermal injection, for a patch, for a pump, or for a depot.

The slow release formulation can be administered by applying, implanting, inserting or injecting a composition herein (*e.g*., injectable microspheres loaded with a bile salt in sustained release form) into a site at or adjacent to a target site to provide treatment. In some embodiments, the composition herein is administered to a target site using *in situ* gel implantation. In some embodiments, the pharmacologically active detergent(s) are entrapped in a polymer carrier such as, but not limited to, poly(DL-lactide-co-glycolide); poly(lactide-co-glycolide); poly(DL-lactide); poly(L-lactide); poly(glycolide); poly(ε-caprolactone); poly(DL-lactide-co-caprolactone).

### Unit Dose

The present invention also contemplates unit doses of the compositions herein. Such unit doses are preferably in a container, a syringe or a syringe loadable container. Such unit doses can have, for example, a total volume of up to 500, 400, 300, 200, 100, 90, 80, 70, 60, 50, 40, 30, 20, 10, 9, 8, 7, 6, 5, 4, 3, 2, 1, 0.9, 0.8, 0.7, 0.6, 0.5, 0.4, 0.3, 0.2, 0.1, 0.09, 0.08, 0.07, 0.06, 0.05, 0.04, 0.03, 0.02, 0.01, 0.009, 0.008, 0.007, 0.006, 0.005, 0.004, 0.003, 0.002, 0.001, 0.0009, 0.0008, 0.0007, 0.0006, 0.0005, 0.0004, 0.0003, 0.0002, or 0.0001 mL. In some embodiments, a unit dose has a total volume in the range of 0.0001-500, 0.0005-400, 0.001-300, 0.005-200, 0.01-100, 0.05-90, 0.06-80, 0.07-70, 0.08-60, 0.09-50, 0.1-40, 0.2-30, 0.3-29, 0.4-28, 0.5-27, 0.6-26, 0.7-25, 0.8-24, 0.9-23, 10-22, 11-21, 12-20, 13-19, 14-18, or 15-17 mL per target site. Other embodiments contemplate a unit dose with a total volume in the range of 0.01-30, 0.02-20, 0.03-10, 0.4-5 or 0.5-1 mL total volume. In some embodiments, a unit dose has a total volume greater 0.0001, 0.0002, 0.0005, 0.001, 0.002, 0.005, 0.01, 0.02, 0.05, 0.1, 0.2, 0.5, 1, 2, 5, 10, 20, 50, 100 mL. Preferably, a unit dose has a total volume of up to 1.0, 0.8, 0.6, 0.4, or 0.2.

A unit dose of the composition herein comprises, consists essentially, or consists of an amount of the one or more pharmacologically active detergents (*e.g*., bile salts, or more preferably salts of deoxycholic acid) in a therapeutically effective amount. Such amount can be determined by a person of ordinary skill in the art and will depend, in part, on the localization of the fat deposit, size of the fat deposit and concentration of active agent(s).

In some embodiments, a unit dose includes up to 10, 9, 8, 7, 6, 5, 4, 3, 2, 1, 0.9, 0.8, 0.7, 0.6, 0.5, 0.4, 0.3, 0.2, 0.1, 0.09, 0.08, 0.07, 0.06, 0.05, 0.04, 0.03, 0.02, 0.01, 0.009, 0.008, 0.007, 0.006, 0.005, 0.004, 0.003, 0.002, 0.001, 0.0009, 0.0008, 0.0007, 0.0006, 0.0005, 0.0004, 0.0003, 0.0002, or 0.0001 grams of the pharmacologically active detergents. In some embodiments, a unit dose includes a range of approximately 0.00001 to 10, 0.00005 to 1.0, 0.0001 to 0.5, 0.0005 to 0.1, 0.001 to 0.05, or 0.005 to 0.01 grams. Preferably a unit dose comprises about 0.01 grams of a bile salt (e.g., sodium deoxycholate).

### Uses

The compositions herein can be used to prevent or reduce the appearance of a skin condition, prevent or reduce the symptoms of sleep apnea, and prevent or reduce the appearance or effects of an adipose condition in a subject. Such subject can be an animal, more preferably a mammal, more preferably a primate (*e.g*., a monkey, chimpanzee, etc.), a domesticated animal (*e.g*., a dog, cat, horse, etc.), a farm animal (*e.g*., goat, sheep, pig, cattle, etc.), a laboratory animal (*e.g*., mouse, rat, etc.), or more preferably a human.

Also disclosed are compositions which can be used to prevent or reduce the appearance of a skin condition selected from the group consisting of loose skin, skin aging, irregularities of the skin, and wrinkles. Such methods involve administering locally to a skin region of interest a composition herein disclosed. The one or more detergents are administered in a skin-tightening effective amount. Up to 5, 4, 3, 2, 1, 0.5, 0.4, 0.3, 0.2, 0.1, 0.05, 0.04, 0.03, 0.02, or 0.01 grams of detergent(s) are administered. A composition may include up to 5, 4, 3, 2, 1, 0.5, 0.4, 0.3, 0.2, 0.1, 0.05, 0.04, 0.03, 0.02, or 0.01 % w/w, w/v or v/v of detergent(s). When one or more lipids are optionally included, such lipids can be phospholipids or more preferably phosphatidylcholine. The mass or volume ratio (e.g., % w/w, w/v or v/v) of detergent(s) to lipid(s) in the composition delivered is preferably greater than 1. A composition may comprise up to 5, 4, 3, 2, 1, 0.5, 0.4, 0.3, 0.2, 0.1, 0.05, 0.04, 0.03, 0.02, or 0.01 % w/w, w/v or v/v lipids, phospholipids or phosphatidylcholine. The compositions being administered are preferably in an aqueous solution. Such solution preferably has a total volume up to 500 mL, 100 mL, 50 mL, 10 mL or 5 mL.

The above compositions are preferably administered locally to a target area to create an inflammatory response causing a scar formation. The formation of a scar results in skin tightening especially in areas where the skin is under little or no tension and provides little resistance to scar contraction. Such treatment may be relevant in a number of clinical scenarios such as, for example, commonly performed fat treatments, including, but not limited to, large volume liposuction. The latter may be associated with post-treatment skin laxity (loose skin in areas of fat removal) and skin surface irregularities. Thus, a composition comprising a therapeutically effective amount of detergent(s) or bile salt(s) may be administered to a liposuction site after completion of the liposuction procedure.

The target area may be an area under eye, under chin, under arm, buttock, calf, back, thigh, stomach, cheek, brow, or any other skin regions showing aging, wrinkles, loose skin or skin irregularity.

The compositions maybe delivered to the target area via a subdermal injection, a pump, a patch, or a subdermal depot. The compositions may be administered topically.

In one aspect, the compositions herein are used for reducing a subcutaneous fat deposit in a subject. The subcutaneous fat deposit can be associated with an adipose conditions such as, for example, obesity, fat redistribution syndrome, eyelid fat herniation, lipomas, lipodystrophy (including buffalo hump lipodystrophy), dorsocervical fat, visceral adiposity, breast enlargement, hyperadiposity, diffused body fat around trunk and arms, fat deposits associated with cellulite, Dercum's disease, Madelung's neck, lipedema, piezogenic nodules, launois cleret syndrome and xanthelasma. The subcutaneous fat deposit can be located in an area of the body, including, but not limited to, under eye, under chin, under arm, buttock, calf, back, thigh, stomach, cheek, brow, "love-handles", ankles, fingers, lips, trachea, etc.

The subcutaneous fat deposits can be reduced by administering locally to the fat deposits a composition consisting essentially of: (i) between 0.05 and 1% w/w of a bile salt, more preferably deoxycholic acid or salts thereof, or more preferably sodium deoxycholate; (ii) a pharmaceutical excipient; wherein the solution is free of phosphatidylcholine or phospholipids. Preferably, the composition does not include lipase or colipase. The above method preferably does not include performing surgery (e.g., liposuction) on the subject.

In any of the above embodiments, the composition being administered is preferably in a solution or more preferably an aqueous solution. Such solution preferably has a total volume up to 500 mL, 100 mL, 50 mL, 10 mL or 5 mL.

The subcutaneous fat deposit can be associated with an adipose condition selected from the group consisting of obesity, fat redistribution syndrome, eyelid fat herniation, lipomas, Dercum's disease, lipodystrophy, buffalo hump lipodystrophy, dorsocervical fat, visceral adiposity, breast enlargement, hyperadiposity, diffused body fat around trunk and arms, and fat deposits associated with cellulite. Thus, the compositions herein can be used to treat or ameliorate conditions associated with such conditions. In some embodiments, the composition herein are administered locally under eye, under chin, under arm, buttock, calf, back, thigh, or stomach of said subject to reduce a subcutaneous fat deposit in such site.

For example, in some embodiments, a composition herein (*e.g*., a bile salt, or more preferably sodium deoxycholate) is used to treat or ameliorate lipodystrophy conditions in a subject, such as a human HIV patient. Lipodystrophy is a condition that is often a side effect from HIV treatments (*e.g*., treatment with protease inhibitors). Lipodystrophy is characterized by regional or generalized loss of subcutaneous fat or abnormal fat redistribution and metabolic disturbances. Signs of lipodystrophy can include a swollen belly along with loss of tissue from the face, arms and legs, which can give an appearance of sunken eyes and sticking-out cheekbones. Other signs of lipodystrophy include fat accumulates on the back of the neck, which is sometimes referred to as buffalo humps (diffused fat accumulation). Female HIV patients may also be treated for breast enlargement which is attributed to the lipodystrophy syndrome.

In some embodiments, the compositions herein are used to treat lipomas. Lipomas are localized fat accumulations that are benign neoplastic growth. There are various forms of lipomas and, in some embodiments; the compositions herein are used to treat multiple familial lipomatosis.

In some embodiments, the compositions herein are used to prevent, treat or ameliorate an adipose in an animal such as a cat, a dog or a horse.

In some embodiments, the compositions herein are used to treat obstructive sleep apnea. Obstructive sleep apnea is characterized by repetitive pauses in breathing during sleep due to the obstruction and/or collapse of an upper airway (throat), usually accompanied by a reduction in blood oxygen saturation, and followed by an awakening to breathe. It is a dangerous (sometimes life threatening) condition that often affects obese people. Obese people have a large amount of fat around their trachea, and this fat may cause their airway to collapse when their muscles relax during sleep. In one embodiment, the compositions herein (*e.g*., sodium deoxycholate) are used to treat obstructive sleep apnea by dissolving fat around the trachea. In such embodiments, the composition herein is administered locally (*e.g*., via injection) to a target site of fat around the trachea in a therapeutically effective amount.

For the treatment of an adipose condition, the compositions herein (*e.g*., pharmacologically active detergent(s), bile salt(s), or sodium deoxycholate) are preferably administered locally to the site of fat accumulation. Localized delivery can be made via, *e.g.*, subcutaneous or transdermal injection, external or internal pump, dermal patch, subcutaneous depot, or any other means known in the art.

Compositions may be delivered via a dermal patch. A dermal patch is a self-adhesive unit, and is worn on a patient's body. It delivers small doses of a drug into the skin, where it then diffuses into the skin. The patch incorporates a series of thin, flexible films: a backing layer, a drug reservoir, a rate-controlling film and an adhesive. Enhancers may be added to further increase drug permeation through the skin. The patch can be coupled with a low-level electrical energy to actively transport drugs through intact skin.

Compositions may be delivered via an external or internal pump. A pump is a specialized device, which delivers drug into the body via a small catheter. For example, an infusion pump can be programmed to deliver drugs at precise dosages and delivery rates. These pumps may have a feedback device that controls drug delivery according to need. A size of the pump depends on an amount of a drug and intended length of treatment.

Compositions may be delivered via a depot. A depot is a non-biodegradable and an osmotically driven implant which is used to enable delivery of drugs for therapy. Powered by osmosis, the depot incorporates a miniature metal alloy cylinder and can provide continuous drug delivery from days to up to one year. Compositions may be administered parenterally. Parenteral routes of administration involve injections into various compartments of the body such as but not limited to, intravenous, subcutaneous, intramuscular, intraperitoneal and the like.

Two injections of up to 1 mL each may be administered under the chin wherein each injection comprises between 0.005 and 0.5, or more preferably between 0.002 and 0.08 grams sodium deoxycholate. Alternatively, 3 treatments of 2 mL 1% w/w sodium deoxycholate may be administered to a cheek area.

A therapeutic regimen can include administering one or more unit doses to a target site. A target site can be for example 0.1 cm², to about 5 cm². The compositions herein maybe administered at the same, adjacent, or nearby target sites at various intervals, dosages, volumes, as disclosed herein. When delivered, the compositions can be administered at various levels below the dermis, including, for example, 0.1-4, 0.2-3.5, 0.3-3, 0.4-2.5, or 0.5-2 inches below the dermis.

For example, up to 100, 10, 9, 8, 7, 6, 5, 4, 3, 2, 1, 0.9, 0.8, 0.7, 0.6, 0.5, 0.4, 0.3, 0.2, 0.1, 0.09, 0.08, 0.07, 0.06, 0.05, 0.04, 0.03, 0.02 or 0.01 mL of a solution may be delivered locally to a target site (*e.g*., a site of fat accumulation or loose skin) at a time. In some embodiments, the compositions (*e.g.,* solutions) herein include no lipase or no colipase, or no colipase and lipase. In some embodiments, the compositions (*e.g*., solutions) herein include no enzymes. Up to 10, 9, 8, 7, 6, 5, 4, 3, 2, 1, 0.9, 0.8, 0.7, 0.6, 0.5, 0.4, 0.3, 0.2, 0.1, 0.09, 0.08, 0.07, 0.06, 0.05, 0.04, 0.03, 0.02 or 0.01 grams of the detergent(s) herein (preferably bile acids or bile salts) are administered locally to a target site at a time. More than 0.001, 0.002, 0.003, 0.004, 0.005, 0.006, 0.007, 0.008, 0.009 or 0.01g can be delivered to a target site at a time. Generally, the total volume, unit dose and number of treatments administered will vary depending on the amount of fat in a target site, the location of the target site, type of fat composition, and desired results. In general, the greater is the amount of fat being treated, the greater is the dose that is administered. Also, the greater the amount of loose skin in a target area, the greater the dose or the more number of injections that will be delivered. It should be noted that while the compositions and unit dosages herein may be administered into an individual as part of a treatment regimen, they are not necessarily actively removed from the individual as part of the treatment regimen (*e.g*., via suction).

A therapeutic regimen can include administration to a target site at least 1, 2, 3, 4, 5, 6, 7, 8, 9, or 10 unit doses. Such unit doses can be delivered simultaneously or over a period of time. For example, administration can occur once, twice, three or four times an hour, a day, a week, a month, or a year. Multiple administrations may involve between 1-100, 2-50, 3-30, 4-20, or 5-10 administrations to a target site an hour, a day, a week, a month or a year. Multiple administrations involve up to 10, 9, 8, 7, 6, 5, 4, 3, or 2 administrations to a target site a year, a week, a day or an hour. The total number of administrations in a therapeutic regimen can be completed with a period of 1 year, 6 months, 5 months, 4 months, 3 months, 2 months, 1 month, 3 weeks, 2, weeks, or 1 week or less.

The methods disclosed herein do not include the surgical removal of one or more localized fat deposits in a subject. A non-surgical method of fat reduction does not include liposuction, lipoplasty or suction lipectomy. For example, the non-surgical methods may not include liposuction. The methods may also exclude non-invasive means for reducing fat, *e.g.*, ultrasonification. Non-invasive means can be used in conjunction with the compositions herein.

Any of the treatments disclosed may be supplemented by further administering to the patient additional active agent(s). Such additional agent(s) can be administered separately or in combination with the compositions herein. A second agent can be administered locally or systemically. Second agents may be administered prior to, simultaneous with, or after the administration of the detergents.

### Kits

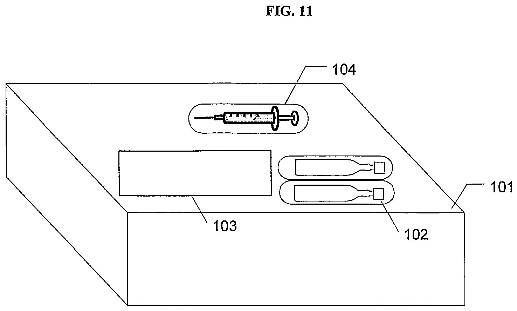

Figure 11 is an illustration of a kit 101 for non-surgical reduction of subcutaneous fat and/or tightening of loose skin. Kit 101 includes one or more containers 102. For example, a first container 102 can include a therapeutically effective amount of a pharmacologically active detergent (*e.g*., bile acids and salts thereof) and up to 5% w/v phosphatidylcholine and a second container 102 can include a therapeutically effective amount of a pharmacologically active detergent and no phosphatidylcholine. Containers 102 are preferably syringe loadable. Containers 102 can each hold one or more unit doses. For example, a container 102 can hold up to 500, 100, 20, 10, 5 or 1 mL.

A container 102 can include one or more additional active ingredients, either independently of or in combination with the one or more detergents herein. Examples of additional active ingredients include antimicrobial agents, anti-thrombotic agents, anti-coagulation agents, suds-depressants, anti-inflammatory agents, analgesics, anesthetics, anti-dispersion agents, dispersion agents, penetration enhancers, steroids, tranquilizers, muscle relaxants, and anti-diarrhea agents.

In one example, a container 102 includes up to 5% w/v of phospholipids (*e.g*. phosphatidylcholine) or no phospholipids (*e.g*. phosphatidylcholine) and up to 10, 5.0, 1.0, or 0.5% w/w detergent(s).

In one example, a container 102 contains more than 0.01, 0.1, 1.0, 2.0, 3.0, 4.0, or 5.0% w/w, w/v or v/v pharmacologically active detergent(s) (*e.g*., bile acids or bile salts) and up to 5% w/v phospholipids (*e.g*. phosphatidylcholine) or no phospholipids (*e.g*. phosphatidylcholine).

The solution of container 102 is administered according to the instructions for use 103. Instructions for use 103 can provide dosing instructions which may depend upon, for example, location of target site, animal being treated, desired results, size of target site, concentration of detergent(s) in composition, etc. Preferably instructions for use 103 are for the treatment of an animal such as a human, a dog, a cat, or a horse. Instructions for use 103 can also include information for treatment of other domesticated animals and/or farm animals. Instruction for use 103 may also include information on the use of the compositions herein to treat specific target sites, such as, *e.g.*, fat deposits or areas of loose skin localized under eye, under chin, under arm, buttock, cheek, brow, calf, back, thigh, ankle, or stomach. Instruction for use 103 may detail an explanation for use of the compositions herein to treat a fat condition of obesity, fat redistribution syndrome, dorsocervical fat, visceral adiposity, breast enlargement, hyperadiposity, eyelid fat herniation, lipomas, lipodystrophy, buffalo hump lipodystrophy, diffused body fat around trunk and arms, or fat deposits associated with cellulite.

Instructions for use 103 may detail an explanation for use of the compositions herein to treat a skin condition selected from the group consisting of loose skin, skin aging, irregularities of the skin, and wrinkles.

Instruction for use 103 may include information regarding proper diluents and volumes for dilution, if any, of the container 102. The instructions for use 103 may also provide information regarding the proper administration of the compositions herein, such as frequency and dosage of administration.

Kit 101 can comprise alternatively or in addition to container 102 one or more syringes 104 or other suitable delivery devices (*e.g*., patches, subcutaneous depots) for delivering the compositions herein (*e.g*. those in container 102) to a target site of fat accumulation or loose skin. A syringe or other delivery device 104 may be preloaded with one or more unit doses of the compositions herein.

Preferably, a kit includes one or more syringes for local subcutaneous injection of a solution having total volume up to 100 mL containing between 0.1-10% of a bile acid or a salt, or more preferably deoxycholic acid or salt thereof or sodium deoxycholate. The solution preferably does not contain lipase or colipase or both. The solution preferably contains up to or no phosphatidylcholine.

Also disclosed are kits having a first container comprising a pharmacologically active detergent and up to 5% w/v phosphatidylcholine, as well as written instructions for reducing subcutaneous fat deposits in a mammal without the use of surgery. Preferably, the kits may be used to reduce fat deposits in a variety of mammals such as, for example, a human, a horse, a dog, or a cat. The mammal may be a human.

The first container may have a total volume of up to 500 mL and/or may be provided as an injectable formulation. The first container may contain a % w/v of detergent greater than the % w/v of phosphatidylcholine or may contain no phosphatidylcholine. The detergent may be at a concentration above its critical micellar concentration (CMC). The kits may comprise a variety of pharmacologically active detergents such as, for example, a lipophilic detergent, a hydrophilic detergent, an ionic detergent, a non-ionic detergent, a glyceride, a bile salt, and a zwitterionic detergent. The active detergent may be a bile salt, most preferably sodium deoxycholate. A first container in the kit may include up to 3 g detergent. A first container in the kit may include more than 0.0002 g detergent. The first container may further include a second detergent.

Preferably, the first container may further comprise a second therapeutic agent such as, for example, an anti-microbial agent, a vasoconstrictor, an anti-thrombotic agent, an anti-coagulation agent, a suds-depressant, an anti-inflammatory agent, an analgesic, a dispersion agent, an anti-dispersion agent, a penetration enhancer, a steroid, a tranquilizer, a muscle relaxant, and an anti-diarrhea agent. The second therapeutic agent may be an analgesic, anti-microbial agent, or an anti-inflammatory agent. More preferably, the second therapeutic agent is an analgesic, or most preferably lidocaine. The kit may provide a second container comprising the second therapeutic agent as described herein.

Also disclosed are kits for reducing fat deposits under the eye, chin, or arm, as well as the buttock, calf, back, thigh, ankle, or stomach of a mammal. The kit may reduce specific types of fat deposits such as, for example, eyelid fat herniation, lipomas, lipodystrophy, buffalo hump lipodystrophy, diffused body fat around trunk and arms, or fat deposits associated with cellulite.

### Business Methods

The methods and the kits disclosed herein can be used to perform business services and/or sell business products.

Also disclosed is a business method that sells the kits disclosed herein or provides treatment services. For example, the business can make a formulation based on the compositions described herein. The business methods herein can then manufacture kits containing such formulations and sell such kits in exchange for a fee. The business method may license a third party to manufacture the kit. The business may contract a sales support to sell such kits.

The business can alternatively or in addition perform treatment services in exchange for service fees. The service can be provided directly to patients and the fee can vary depending on the length of the procedure and/or amount of active detergents used.

The following examples are meant to illustrate one or more embodiments of the invention and are not meant to limit the invention to that which is described below.

### EXAMPLES

### Example 1 (not according to the invention)

### Sodium deoxycholate and phosphatidylcholine formulations

Phosphatidylcholine bile salt formulation (PBF) (5.0% highly purified soy derived PC, 4.75% sodium deoxycholate, and 0.9% benzyl alcohol, in sterile water, Table 2) was obtained from Hopewell Pharmacy, Hopewell, NJ. Sodium deoxycholate and Triton X-100 detergent (Triton, alkylaryl polyether alcohol) were obtained from Sigma-Aldrich Corp. (St. Louis, MO). Empigen BB detergent (Empigen, lauryldimethylbetaine) was obtained from Calbiochem, Biosciences, Inc., (La Jolla, CA). An injectible PBF solution was made according to Table 2 below. Stock reagents (5% dilutions) were prepared in PBS buffer.

**Table 2. Injectable PBF**

| | |
|---|---|
| Phosphatidylcholine | 5.00 % (w/v) |
| Sodium deoxycholate | 4.75 % |
| Benzyl alcohol | 0.90 % |
| Water | 100 mL |

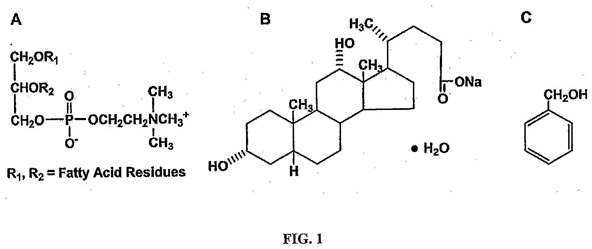

The molecular structure of (a) phosphatidylcholine, (b) sodium deoxycholate and (c) benzyl alcohol are depicted in FIG. 1.

### Example 2 (the use of PBF is not according to the invention; only 0.05-1% deoxycholate formulations are according to the invention)

### Effects of sodium deoxycholate and phosphatidylcholine solutions in cultured cells

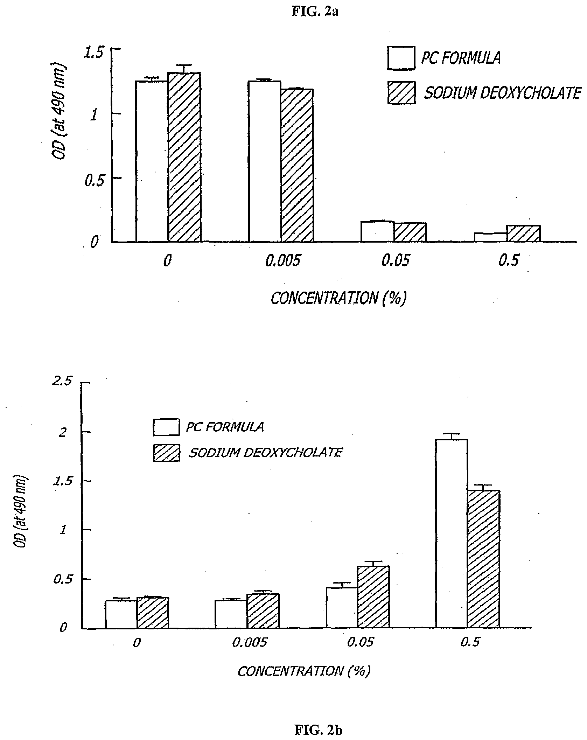

To measure cell viability after detergent treatment, HaCaT human keratinocyte cells were cultured in DMEM (Dulbecco's modified Eagle's medium) supplemented with 10% fetal calf serum, penicillin, and streptomycin. HaCaT cells were cultured in 6 well plates and incubated with 0, 0.005, 0.050 or 0.500% of the PBF or sodium deoxycholate for 30 min at 37°C prior to determination of cell viability using the MTS assay, which uses a tetrazolium compound that produces a color change when bioreduced by metabolically active cells (CellTiter 96 Aqueous Non-Radioactive Cell Proliferation Assay, Promega, Corp. Madison, WI). Cell viability was determined by an absorbance spectrophotometer (at 490 nm) after 4 hour incubation with the assay at 37°C. To determine cell viability in fresh tissue, fat specimens were incubated for 4 hours in 24 well plates with stock reagents and the MTS assay. Tissue specimens were then visualized for color change and the amount of MTS in their supernatants was measured by absorbance (at 490 nm). All studies were performed in triplicate. Absorbance at 490 nm (OD 490) is proportional to the number of living cells in the culture. There was comparable OD 490 in the control and 0.005% dilutions of both compounds (FIG. 2a), indicating little effect of these substances on cell viability at this concentration. Cell viability progressively decreased at 0.05% and 0.5% concentrations of both solutions.

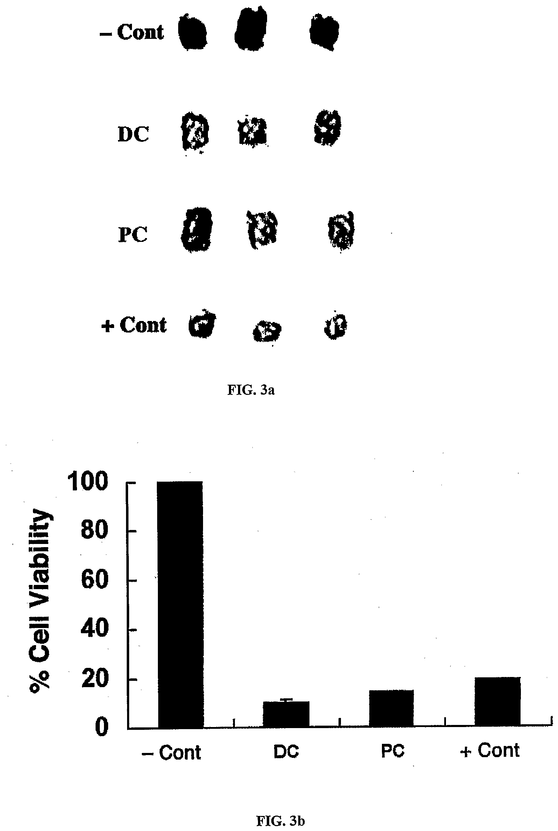

Cell lysis in response to detergent treatment was determined in HaCaT cells incubated with the reagents at the indicated cell dilutions for 30 min at 37°C. Lysis in cultured cells was measured using a lactate dehydrogenase (LDH) assay and within tissue using calcein, a fluorescent marker retained in cells with intact cell membranes. The LDH assay measures the activity of LDH, which is a cytosolic enzyme released when cells are lysed. Lactate dehydrogenase release was measured by absorbance (at 490 nm) after a 1 hour incubation with the LDH assay as recommended by the manufacturer (CytoTox 96 Non-Radioactive Cytotoxicity Assay, Promega). All studies were performed in triplicate. LDH release is directly proportional to absorbance at 490 nm (OD 490). Because penetration into intact tissues may be likely a limiting factor, cell cultures were used to determine the dilutions of the reagents (PBF and deoxycholate) necessary to affect cells. As is illustrated in FIG. 2a, sodium deoxycholate profoundly decreased the viability of cultured cells approximately equal to the complete PBF. This finding was reproduced in tissue by exposing porcine fat to PBF and deoxycholate (FIG. 3). These results support the unexpected observation that sodium deoxycholate plays a major, active role in the PBF. There was minimal LDH release from control cells and those incubated with 0.005% dilutions of both compounds (FIG. 2b). Moreover, both the PBF and deoxycholate treated cell cultures demonstrated a concentration dependent increase in cell lysis (FIG. 2b). Moreover, the direct lytic effects observed in cultured cells treated with these agents suggest activity independent of endogenous lipase. There was progressively more LDH released at 0.05% and 0.5% of the PBF and deoxycholate. Comparing the effects of the PBF to deoxycholate in cell culture led to the surprising result that deoxycholate caused similar loss of cell viability, but less cell lysis. These data unexpectedly demonstrate that deoxycholate acts as the active component in PBF.

### Example 3 (PBF and 5% solutions of deoxycholate are not according to the invention)

### Effects of sodium deoxycholate and phosphatidylcholine solutions in porcine tissue

Porcine tissue was obtained immediately after sacrifice, shaved, and placed on ice for a maximum of four hours before use. Fat specimens were obtained by removing the epidermis and dermis of a punch biopsy with a scalpel and trimmed. Fat specimens were loaded with calcein dye by incubating 1 hour at 37°C with Calcein-AM (Sigma). Stock reagents were added to the fat specimens and incubated for 30 min at 37°C with gentle agitation. Calcein retention was determined by tissue fluorescence using purple (411 nm) light and visually observing the emitted green (500 nm) light using an emission filters.

Histology was performed by injecting stock reagent solutions (0.5 mL) into full thickness porcine skin at various levels (epidermis, dermis, and subcutaneous tissue) with 1.0 mL syringes and 30-gauge, 0.5 inch needles. Needle depth was visualized along the margin of the porcine tissue with the intent of saturating the target tissue. One hour after incubation with PBS at 37°C, multiple 5.0 mm biopsy specimens were obtained from the injected sites, each condition performed in triplicate. Tissue was fixed in formaldehyde, paraffin-embedded, and stained with hematoxylin-eosin. Specimens were evaluated by a board-certified dermatopathologist who was blinded to the treatment protocol.

Fresh porcine skin was used to determine if the effects of these detergent substances on cultured cells were similar in tissue. FIG. 3a demonstrates the production of dark purple pigment (indicating viable cells) in fat tissue treated with the PBS buffer (negative control) using the MTS assay. The PBF and 5% solutions of deoxycholate and Triton detergent (positive control) demonstrated a comparable loss of purple dye (indicating cell death) in the treated fat specimens. The difference in fat cell viability between the solutions was quantified by measuring the absorbance (at 490) of the supernatants collected from the treated fat specimens (FIG. 3b). All reagents had significant effects on the fat cell viability of fresh tissue.

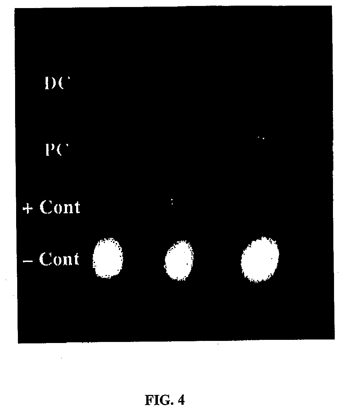

Cell lysis was confirmed using a calcein dye release assay. Calcein becomes fluorescent after hydrolysis and is retained in cells that have intact cell membranes. Because it does not label dead cells and is lost under conditions that cause cell lysis, loss of green fluorescence in fat tissue samples loaded with the dye calcein indicates cell lysis (FIG. 4). Samples treated with the deoxycholate, PBF, and Triton detergent (positive control) exhibited similar loss of fluorescence.

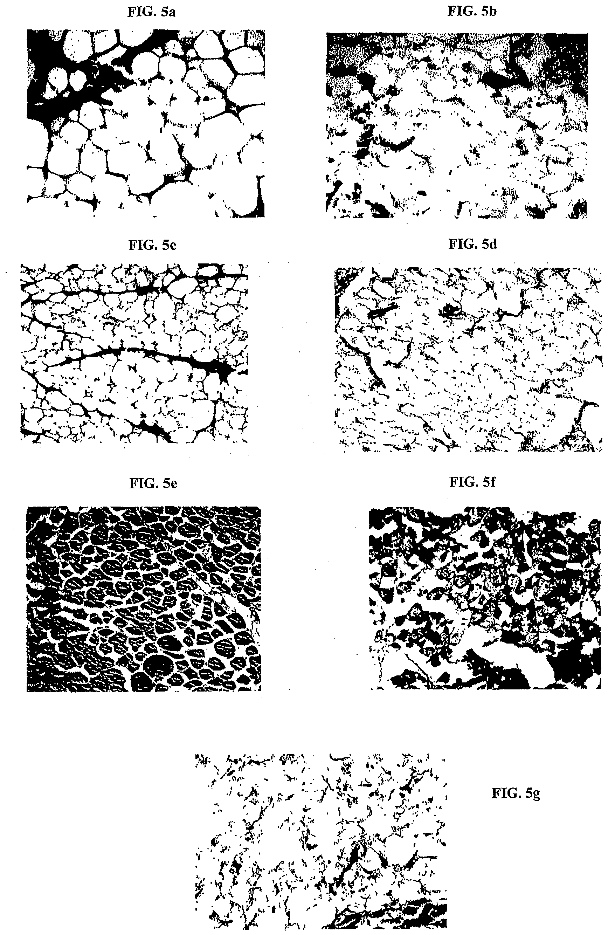

The histologic changes resulting from injection of PBF, deoxycholate, and Empigen (also not according to the invention) are shown in FIG. 5. Phosphatidylcholine bile salt formulation (FIG. 5b) and deoxycholate (FIG. 5d) produced histologic effects similar to those caused by Empigen (FIG. 5g) and Triton (not shown), two well-characterized laboratory detergents. These changes were apparent in both fat and muscle. Marked blurring and dissolution of adipocyte cell membranes with disruption of its normal lobular architecture were seen, after injection of both the PBF (FIG. 5b) and deoxycholate (FIG. 5d). FIG. 5f demonstrates muscle fiber disarray and atrophy after PBF injection. Similar changes in muscle tissue were visible in the specimens treated with deoxycholate and the Triton and Empigen detergents. There were no changes in the epidermis, dermis, or adnexal structures after injection of the reagents with the exception of Empigen, which caused loss of fibroblast nuclear staining and hyalinization of dermal collagen. Moreover, from clinical reports, it is apparent that a brisk inflammatory response, such as erythema and edema, occurs after injection with the detergents. Repeated inflammation can potentially lead to fibrosis, especially after multiple injections. Fibrosis has been reported in several patients who developed firm nodules at injection sites after PBF administration that eventually resolve over several months.

Histologic findings reveal that the injectable PBF and deoxycholate alone cause architectural disruption in fat and muscle, but had no apparent effect on the epidermis, dermis, or adnexae (FIG. 5). However, Empigen BB, a potent laboratory detergent, had profound histologic effects on dermal collagen (connective tissue). Alternatively, fat and muscle can be more sensitive to detergent treatment than these other structures at the tested concentrations (similar to those used in clinical practice).

Through a series of laboratory experiments utilizing fresh tissue specimens and cell cultures, it has been demonstrated that the PBF popularly used in subcutaneous injections for fat dissolution works primarily by causing non-specific lysis of cell membranes. Cell membranes are constituents of all tissue types; specifically, the present inventor demonstrated that these detergents cause solubilization of fat, muscle and connective tissue. Therefore sodium deoxycholate, the bile salt component of the formula used to dissolve the phosphatidylcholine, was the major active ingredient of these prior art formulations. This conclusion is supported by the fact that pharmacologically active detergents, such as bile salts are potent solubilizers of cell membranes.

### Example 4 (2.5 and 5% solutions of deoxycholate are not according to the invention)

### Clinical Experience with sodium deoxycholate compositions

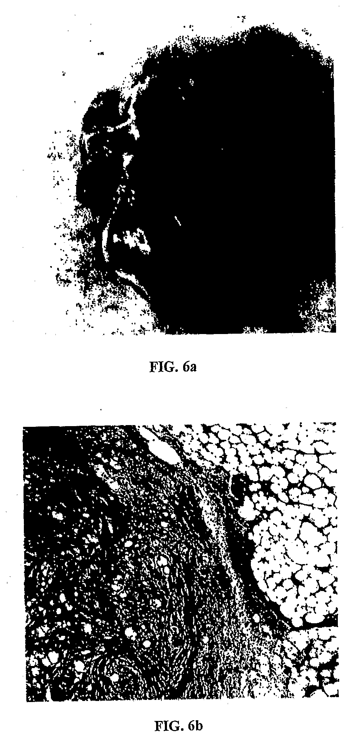

Patients having lipomas, benign, isolated collections of adipose tissue, were injected with sodium deoxycholate (DC) solutions without phosphatidylcholine directly into the lipoma. The results of this study demonstrate that the detergent effects of deoxycholate seen on fat in animal tissues are reproducible clinically in humans. All injected lipomas were reduced in size after at least one treatment with varied concentrations of deoxycholate (Table 3). A lipoma from one patient, injected with 1% DC, was excised after treatment and pathological and histological analysis performed. Within the excised lipoma, necrosis is visible grossly (FIG. 6a) with a well demarcated area of hemorrhage and necrosis on the lateral edge extending into the middle of the lipoma fat which contrasts with the normal lipoma fat which is lighter in color. Histological analysis (FIG. 6b) reveals a well defined area of hemorrhage and necrotic fat as well as a significant inflammatory reaction which contrasts to the adjacent normal round clear fat cells.

**Table 3. Reduction in size of lipomas after DC treatment**

| **Lipoma** | **Size (cm) Pre-treatment** | **Size(cm) Post-treatment** | **Total Treatments (% DC injected)** |
|---|---|---|---|
| 1 | 2.00 x 1.00 | 1.25 x 0.50 | 2 (2.5%) |
| 2 | 2.00 | 1.50 x 0.50 | 3 (5% and 2.5%) |
| 3 | 2.00 x 2.50 | 2.00 x 1.00 | 3 (5% and 2.5%) |
| 4 | 4.00 x 1.75 | 2.50 x 2.00 | 2 (1%) |
| 5 | 2.00 x 1.75 | 1.25 | 2 (1%) |
| 6 | 2.80 | 0.50 | 1 (5%) |
| 7 | 1.00 | Imperceptible | 1 (1%) |

### Example 5

### Treatment of lipodystrophy in a HIV patient with sodium deoxycholate compositions

An HIV lipodystrophy patient with symmetrical fat deposits in his face just ventral of his master muscle (sort of in his cheeks, like acorns in the mouth of a chipmunk) was injected with 1% deoxycholate. At 1 month follow up, there was a 50% apparent reduction in deposit volume on both sides. The results of this study demonstrate that the effects of deoxycholate seen on fat in animal tissues are reproducible clinically in humans.

### Example 6 (only 0.05-1% deoxycholate formulations are according to the invention; the addition of PC is not according to the invention)

### Effects of sodium deoxycholate and sodium deoxycholate-phosphatidylcholine solutions on melanocytes

Melanocytes in melanocyte media on a 96-well dish were differentiated at 37°C in a humidified, 5% CO₂ incubator for a week. Media was aspirated and washed with 1xPBS once. Cells were treated with different concentrations of DC (from 0 to 0.5%) with or without 1 % PC in 1xPBS. Each treatment was done in triplicates (Table 4). The plates were incubated at 37°C in a humidified, 5% CO₂ incubator for 30 min. The incubated plates were aspirated and washed with 1xPBS.

10 mL of MTS (3-(4, 5-dimethyl-2-yl)-5-(3-carboxymethoxyphenyl)-2-(4-sulfophenyl)-2H-tetrazolium, inner salt) assay solution was prepared (mixed 40 uL MTS (Promega, Madison WI) per Mel 1xPBS). 100 uL of MTS solution was added to each well of the plates. The plates were incubated at 37°C incubator (non-humified) for 120 min. OD was taken with spectrophotometer at 490 nm.

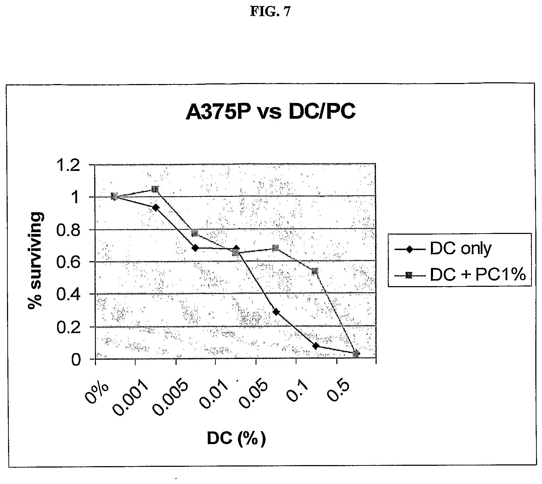

FIG. 7 illustrates the survival of melanocytes in sodium deoxycholate solution and sodium deoxycholate with 1% phosphatidylcholine solution. A375P is a melanoma cell line (P represents poorly metastatic). On y-axis, 1 represents 100% survival when no DC is added. All % concentrations are w/v%. LD50 (concentration at which 50% of the fat cells are dead) of only sodium deoxycholate solution for melanocyte cell line was found to be around 0.04% sodium deoxycholate. The addition of sodium deoxycholate with 1% phosphatidylcholine solution increases the LD50 by ∼5-6 fold.

Results of this study demonstrate that addition of 1% phosphatidylcholine inhibits apoptosis in vitro i.e., the presence of PC makes it 5-times harder for DC to kill the melanocytes, showing that PC detracts from, and thus does not enhance the cell killing power of DC.

**Table 4. Melanocytes after treatment with DC only and DC + 1% PC**

| **DC** | **Melanocytes surviving in DC only** | **Average** | **Percentage surviving%** | **Melanocytes surviving in DC + 1% PC** | **Average** | **Percentage surviving%** |
|---|---|---|---|---|---|---|
| 0% | 0.163 | 0.166667 | 1 | 0.283 | 0.342333 | 1 |
| | 0.16 | | | 0.326 | | |
| | 0.177 | | | 0.418 | | |
| 0.001 | 0.158 | 0.155333 | 0.932 | 0.248 | 0.356333 | 1.040896 |
| | 0.148 | | | 0.239 | | |
| | 0.16 | | | 0.582 | | |
| 0.005 | 0.128 | 0.113333 | 0.68 | 0.226 | 0.263667 | 0.770204 |
| | 0.109 | | | 0.259 | | |
| | 0.103 | | | 0.306 | | |
| 0.01 | 0.124 | 0.112333 | 0.674 | 0.197 | 0.220667 | 0.644596 |
| | 0.107 | | | 0.223 | | |
| | 0.106 | | | 0.242 | | |
| 0.05 | 0.056 | 0.047333 | 0.284 | 0.207 | 0.232 | 0.677702 |
| | 0.038 | | | 0.214 | | |
| | 0.048 | | | 0.275 | | |
| 0.1 | 0.009 | 0.013 | 0.078 | 0.191 | 0.182333 | 0.532619 |
| | 0.014 | | | 0.168 | | |
| | 0.016 | | | 0.188 | | |
| 0.5 | 0.001 | 0.004 | 0.024 | 0.012 | 0.006333 | 0.0185 |
| | 0.009 | | | 0.003 | | |
| | 0.002 | | | 0.004 | | |

### Example 7 (only 0.05-1% deoxycholate formulations are according to the invention; the addition of PC is not according to the invention)

### Effects of sodium deoxycholate and sodium deoxycholate-phosphatidylcholine solutions on adipocytes

Adipocytes in adipocyte media on a 96-well dish were differentiated at 37°C in a humidified, 5% CO₂ incubator for a week. Media was aspirated and washed with 1xPBS once. Cells were treated with different concentrations of DC (from 0 to 0.5%) with or without 1 % PC in 1xPBS. Each treatment was done in triplicates (Table 5). The plates were incubated at 37°C in a humidified, 5% CO₂ incubator for 30 min. The incubated plates were aspirated and washed with 1xPBS.

10 mL of MTS (3-(4, 5-dimethyl-2-yl)-5-(3-carboxymethoxyphenyl)-2-(4-sulfophenyl)-2H-tetrazolium, inner salt) assay solution was prepared (mixed 40 uL MTS (Promega, Madison WI) per 1 mL 1xPBS). 100 ul of MTS solution was added to each well of the plates. The plates were incubated at 37°C incubator (non-humidified) for 120 min. OD was taken with spectrophotometer at 490 nm.

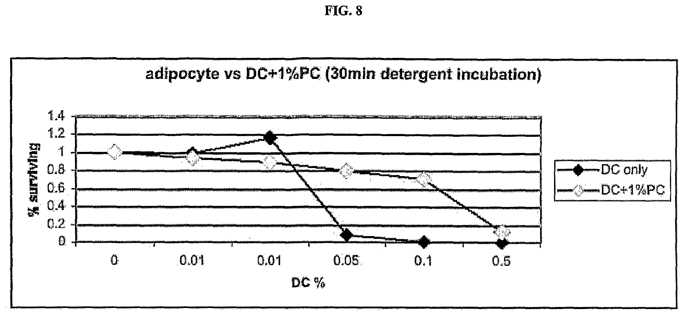

FIG. 8 illustrates the survival of adipocytes in sodium deoxycholate solution and sodium deoxycholate with 1% phosphatidylcholine solution. The adipocyte cells are primary (i.e., taken freshly from a person) adipocytes, cultured in vitro to maturity before treatment with DC. On y-axis, 1 represents 100% survival when no DC is added. All % concentrations are w/v%. LD50 (concentration at which 50% of the fat cells are dead) of only sodium deoxycholate solution for adipocyte cell line was found to be around 0.02% sodium deoxycholate. The addition of sodium deoxycholate with 1% phosphatidylcholine solution increases the LD50 by ∼5-6 fold.

Results of this study demonstrate that addition of 1% phosphatidylcholine inhibits apoptosis in vitro i.e., the presence of PC makes it 5-times harder for DC to kill the adipocytes, showing that PC detracts from, and thus does not enhance the fat cell killing power of DC.

**Table 5. Adipocytes after treatment with DC only and DC + 1% PC**

| **DC** | **Adipocytes surviving in DC only** | **Average** | **Percentage surviving%** | **Adipocytes surviving in DC + 1% PC** | **Average** | **Percentage surviving%** |
|---|---|---|---|---|---|---|
| 0% | 0.036 | 0.113 | 1 | 0.191 | 0.200 | 1 |
| | 0.112 | | | 0.19 | | |
| | 0.114 | | | 0.22 | | |
| 0.005 | 0.52 | 0.111 | 0.982 | 0.195 | 0.186 | 0.930 |
| | 0.105 | | | 0.178 | | |
| | 0.117 | | | 0.186 | | |
| 0.01 | 0.067 | 0.131 | 1.163 | 0.174 | 0.178 | 0.891 |
| | 0.133 | | | 0.183 | | |
| | 0.13 | | | 0.179 | | |
| 0.05 | 0.01 | 0.09 | 0.079 | 0.167 | 0.159 | 0.795 |
| | 0.009 | | | 0.164 | | |
| | 0.008 | | | 0.147 | | |
| 0.1 | 0 | 0 | 0 | 0.138 | 0.141 | 0.707 |
| | 0 | | | 0.14 | | |
| | 0 | | | 0.147 | | |
| 0.5 | 0 | 0 | 0 | 0.032 | 0.024 | 0.123 |
| | 0 | | | 0.02 | | |
| | 0 | | | 0.022 | | |

### Example 8 (not according to the invention)

### Effects of addition of phosphatidylcholine to 4.75% sodium deoxycholate solutions on viable adipocytes

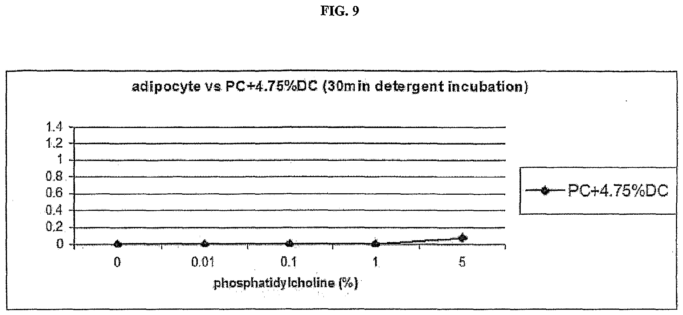

FIG. 9 illustrates survival of adipocytes when phosphatidylcholine is added to 4.75% sodium deoxycholate solution. On y-axis, 1 represents 100% survival. All % concentrations are w/v%. Survival of the cells in the absence of DC and PC is on an average 0.113.

Results of this study demonstrate that addition of phosphatidylcholine to sodium deoxycholate solution does not necessarily contribute to adipolysis in vitro, as deoxycholate is both necessary and sufficient to mediate 100% adipolysis of cultured adipocytes. Furthermore, a close examination of these data (Table 6) shows that 5% phosphatidylcholine actually inhibits adipolysis slightly, which is not surprising in the light of the 5-6 fold increase in LD50 conferred by the addition of phosphatidylcholine (as demonstrated in example 7). These data collectively support the notion that sodium deoxycholate is an active ingredient for adipolysis, and that phosphatidylcholine is not only unnecessary, but actually inhibits fat removal.

**Table 6. Adipocytes after addition of PC to 4.75% DC**

| **PC** | **Adipocytes surviving by adding PC to 4.75% DC** | **Average** | **Percentage surviving%** |
|---|---|---|---|
| 0 | 0 | 0 | 0 |
| | 0 | | |
| | 0 | | |
| 0.01 | 0 | 0 | 0 |
| | 0 | | |
| | 0 | | |
| 0.1 | 0 | 0 | 0 |
| | 0 | | |
| | 0 | | |
| 1 | 0 | 0 | 0 |
| | 0 | | |
| | 0 | | |
| 5 | 0.002 | 0.009 | 0.079 |
| | 0.013 | | |
| | 0.012 | | |

### Example 9

### Inhibition of adipolysis by pre-incubation with human lipoma fat

The experiment was performed on a series of immortalized melanocyte cell lines. Resected human lipomas were ground up, and DC-containing media was mixed with the human fat and agitated for 24 hours. The material was centrifuged to pellet insoluble contents. The supernatants were then added on top of cultured melanocytes. Control was the identical DC-containing media not exposed to the fat chunks. Also, fat alone (no media) was added as a control.

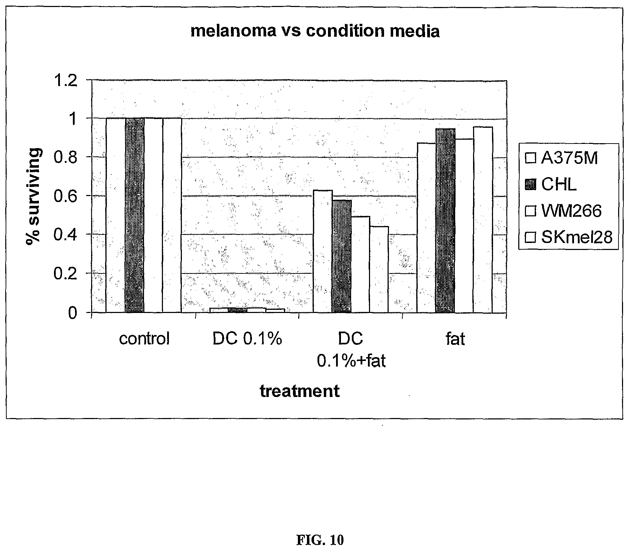

FIG. 10 shows that pre-incubation of 0.1% DC with the fat (Table 7) appears to reduce killing. It could be by releasing some form of inhibitor (e.g., example 7 shows that PC inhibits DC killing in vitro) or could be by retaining the DC in the fat pellet that was spun out in the centrifuge. But it is evident in this experiment that the presence of the fat limits the killing properties of DC, may be by direct inhibition (like PC) or by sequestration (i.e., into the fat pellet). This can further explain the observation that injection of DC into fat spares the surrounding tissues.

**Table 7. Inhibition of adipolysis by pre-incubation with human lipoma fat**

| | **Control** | | | **DC 0.1%** | | | **DC 0.1% + fat** | | | **Fat** | | |
|---|---|---|---|---|---|---|---|---|---|---|---|---|
| A375M | 0.598 | 0.602 | 0.616 | 0.066 | 0.063 | 0.063 | 0.456 | 0.382 | 0.365 | 0.494 | 0.517 | 0.601 |
| Actual | 0.598 | 0.602 | 0.616 | 0.066 | 0.063 | 0.063 | 0.456 | 0.382 | 0.365 | 0.494 | 0.517 | 0.601 |
| Average | | | 0.605 | | | 0.064 | | | 0.401 | | | 0.537 |
| Percentage | | | 1.000 | | | 0.106 | | | 0.662 | | | 0.888 |
| CHL | 0.63 | 0.683 | 0.663 | 0.064 | 0.066 | 0.064 | 0.402 | 0.426 | 0.378 | 0.612 | 0.623 | 0.646 |
| Actual | 0.63 | 0.683 | 0.663 | 0.064 | 0.066 | 0.064 | 0.402 | 0.426 | 0.378 | 0.612 | 0.623 | 0.646 |
| Average | | | 0.659 | | | 0.065 | | | 0.402 | | | 0.627 |
| Percentage | | | 1 | | | 0.098 | | | 0.610 | | | 0.952 |
| WM266 | 0.548 | 0.56 | 0.567 | 0.07 | 0.061 | 0.058 | 0.281 | 0.308 | 0.314 | 0.48 | 0.507 | 0.533 |
| Actual | 0.548 | 0.56 | 0.567 | 0.07 | 0.061 | 0.058 | 0.281 | 0.308 | 0.314 | 0.48 | 0.507 | 0.533 |
| Average | | | 0.558 | | | 0.063 | | | 0.301 | | | 0.507 |
| Percentage | | | 1.000 | | | 0.113 | | | 0.539 | | | 0.907 |
| SKmel28 | 0.583 | 0.643 | 0.620 | 0.061 | 0.060 | 0.060 | 0.290 | 0.304 | 0.302 | 0.569 | 0.591 | 0.616 |
| Actual | 0.583 | 0.643 | 0.620 | 0.061 | 0.060 | 0.060 | 0.290 | 0.304 | 0.302 | 0.569 | 0.591 | 0.616 |
| Average | | | 0.615 | | | 0.060 | | | 0.299 | | | 0.592 |
| Percentage | | | 1.000 | | | 0.098 | | | 0.485 | | | 0.962 |
| Blank | 0.052 | 0.052 | 0.052 | 0.052 | 0.052 | 0.052 | 0.052 | 0.052 | 0.052 | 0.052 | 0.052 | 0.052 |

### Example 10

### Human lipoma studies with sodium deoxycholate compositions

Six patients having 12 lipomas were injected with sodium deoxycholate (DC) solutions over a period of six months. All injected lipomas reduced in size after at least one treatment (Table 8). Measurements were made by physical measurements and ultrasound imaging. The results of this study demonstrate that the detergent effects of deoxycholate seen on fat in animal tissues are reproducible clinically in humans and that there is a significant reduction in the size of lipomas after the treatment.

**Table 8. Human lipoma studies with sodium deoxycholate compositions**

| **Lipoma** | **Pre-treatment size (cm)** | **Post-treatment size** | **Change in volume (%)** | **Total Treatments** |
|---|---|---|---|---|
| 1 | 1 | 0 | -100% | 1 |
| 2 | 2 | 1.0, 0.3, 0.5 | n/a | 3 |
| 3 | 2 x 2.5 | 1 x 1.5 | 84% | 3 |
| 4 | 4 x 3.5 | 2 | 84% | 4 |
| 5 | 2 x 1.8 | 1.3 | 92% | 4 |
| 6 | 2.8 | 0.5 | 99% | 1 |
| 7 | 3 x 2 | 0.8 | 97% | 2 |
| 8 | 1 | 0 | 100% | 2 |
| 9 | 2 x 1 | 1.2 x 0.8 | 67% | 2 |
| 10 | 2 | 1.3 | 73% | 2 |
| 11 | 2 | 1.4 x 1 | 79% | 1 |
| 12 | 1 | 0.8 | 50% | 1 |

Unless otherwise indicated, all numbers expressing quantities of ingredients, properties such as molecular weight, reaction conditions, and so forth used in the specification are to be understood as being modified in all instances by the term "about." Accordingly, unless indicated to the contrary, the numerical parameters set forth in the following specification are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values Size (cm) set forth in the specific examples are reported as precisely as possible. Any numerical value, however, inherently contains certain errors necessarily resulting from the standard deviation found in their respective testing measurements.

The terms "a" and "an" and "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

## Claims

1. A pharmaceutical solution for therapeutic use in the non-surgical removal of one or more localized fat deposits in a subject, wherein the solution is to be administered by subcutaneous injection, said solution consisting essentially of:
between 0.05 and 1% w/w of a bile salt; and
a pharmaceutical excipient;
wherein the solution is free of phosphatidylcholine or phospholipids.

2. The pharmaceutical solution for use as defined in claim 1, wherein said bile salt is the salt of a bile acid selected from the group consisting of: deoxycholic acid, cholic acid, chenodeoxycholic acid, 7-alpha-dehydroxylate chenodeoxycholic acid, lithocholic acid, ursodeoxycholic acid, dihydroxytaurin acid, trihydroxytaurin acid, and glycine conjugates of any of the above.

3. The pharmaceutical solution for use as defined in claim 1 or claim 2, wherein said bile salt comprises a cation selected from the group consisting of sodium (Na⁺), potassium (K⁺), lithium (Li⁺), magnesium (Mg²⁺), calcium (Ca²⁺), barium (Ba²⁺), strontium (Sr²⁺), and ammonium (NH₄⁺).

4. The pharmaceutical solution for use as defined in any one of claims 1 to 3, wherein said bile salt comprises an alkali metal selected from the group consisting of sodium (Na⁺), potassium (K⁺), and lithium (Li⁺).

5. The pharmaceutical solution for use as defined in any one of claims 1 to 3, wherein said bile salt comprises an alkali earth metal selected from the group consisting of magnesium (Mg²⁺), calcium (Ca²⁺), barium (Ba²⁺), and strontium (Sr²⁺).

6. The pharmaceutical solution for use as defined in any one of claims 2 to 5, wherein said bile acid is deoxycholic acid.

7. The pharmaceutical solution for use as defined in any one of claims 1 to 4, wherein said bile salt is sodium deoxycholate.

8. The pharmaceutical solution for use as defined in any one of claims 1 to 7, wherein said solution is an aqueous solution.

9. The pharmaceutical solution for use as defined in claim 7 or claim 8, wherein the solution comprises sodium deoxycholate in a concentration of between 0.05% and 0.5%.

10. The pharmaceutical solution for use as defined in any one of claims 7 to 9, wherein the solution comprises sodium deoxycholate in a concentration of between 0.1% and 0.5%, such as 0.1% or 0.5%.

11. The pharmaceutical solution for use as defined in any one of claims 1 to 10, wherein the solution additionally comprises an aqueous vehicle selected from saline, water, benzyl alcohol and acetic acid.

12. The pharmaceutical solution for use as defined in any one of claims 1 to 11, wherein the localized fat deposit is in a human.

13. The pharmaceutical solution for use as defined in any one of claims 1 to 12, wherein the localized fat deposit is associated with an adipose conditions such as, for example, obesity, fat redistribution syndrome, eyelid fat herniation, lipomas, lipodystrophy (including buffalo hump lipodystrophy), dorsocervical fat, visceral adiposity, breast enlargement, hyperadiposity, diffused body fat around trunk and arms, Dercum's disease, Madelung's neck, lipedema, piezogenic nodules, launois cleret syndrome and xanthelasma.

14. The pharmaceutical solution for use as defined in any one of claims 1 to 13, wherein the solution is delivered to an area of the body selected from: under eye, under chin, under arm, buttock, cheek, brow, calf, back, thigh, ankle, or stomach.

## Patentansprüche

1. Pharmazeutische Lösung zur therapeutischen Verwendung bei der nichtoperativen Entfernung von einer oder mehreren lokalen Fettablagerungen bei einem Subjekt, wobei die Lösung durch subkutane Injektion zu verabreichen ist, wobei die Lösung im Wesentlichen besteht aus:
zwischen 0,05 und 1 Gew.-% eines Gallensalzes; und
einem pharmazeutischen Hilfsstoff;
wobei die Lösung frei von Phosphatidylcholin oder Phospholipiden ist.

2. Pharmazeutische Lösung zur Verwendung, wie in Anspruch 1 definiert, wobei das Gallensalz das Salz einer Gallensäure ist, die aus der Gruppe, bestehend aus:
Desoxycholsäure, Cholsäure, Chenodesoxycholsäure, 7-alpha-deshydroxylierte Chenodesoxycholsäure, Lithocholsäure, Ursodesoxycholsäure, Dihydroxytaurinsäure, Trihydroxytaurinsäure und Glycin-Konjugate von irgendeinem der obigen, ausgewählt ist.

3. Pharmazeutische Lösung zur Verwendung, wie in Anspruch 1 oder Anspruch 2 definiert, wobei das Gallensalz ein Kation umfasst, das aus der Gruppe ausgewählt ist, die aus Natrium (Na⁺), Kalium (K⁺), Lithium (Li⁺), Magnesium (Mg²⁺), Calcium (Ca²⁺), Barium (Ba²⁺), Strontium (Sr²⁺) und Ammonium (NH₄⁺) besteht.

4. Pharmazeutische Lösung zur Verwendung, wie in einem der Ansprüche 1 bis 3 definiert, wobei das Gallensalz ein Alkalimetall umfasst, das aus der Gruppe ausgewählt ist, die aus Natrium (Na⁺), Kalium (K⁺) und Lithium besteht.

5. Pharmazeutische Lösung zur Verwendung, wie in einem der Ansprüche 1 bis 3 definiert, wobei das Gallensalz ein Erdalkalimetall umfasst, das aus der Gruppe ausgewählt ist, die aus Magnesium (Mg²⁺), Calcium (Ca²⁺), Barium (Ba²⁺) und Strontium (Sr²⁺) besteht.

6. Pharmazeutische Lösung zur Verwendung, wie in einem der Ansprüche 2 bis 5 definiert, wobei die Gallensäure Desoxycholsäure ist.

7. Pharmazeutische Lösung zur Verwendung, wie in einem der Ansprüche 1 bis 4 definiert, wobei das Gallensalz Natriumdesoxycholat ist.

8. Pharmazeutische Lösung zur Verwendung, wie in einem der Ansprüche 1 bis 7 definiert, wobei die Lösung eine wässrige Lösung ist.

9. Pharmazeutische Lösung zur Verwendung, wie in Anspruch 7 oder Anspruch 8 definiert, wobei die Lösung Natriumdesoxycholat in einer Konzentration zwischen 0,05% und 0,5% umfasst.

10. Pharmazeutische Lösung zur Verwendung, wie in einem der Ansprüche 7 bis 9 definiert, wobei die Lösung Natriumdesoxycholat in einer Konzentration zwischen 0,1% und 0,5%, zum Beispiel 0,1% oder 0,5%, umfasst.

11. Pharmazeutische Lösung zur Verwendung, wie in einem der Ansprüche 1 bis 10 definiert, wobei die Lösung zusätzlich ein wässriges Vehikel umfasst, das aus Kochsalzlösung (saline), Wasser, Benzylalkohol und Essigsäure ausgewählt ist.

12. Pharmazeutische Lösung zur Verwendung, wie in einem der Ansprüche 1 bis 11 definiert, wobei die lokale Fettablagerung bei einem Menschen ist.

13. Pharmazeutische Lösung zur Verwendung, wie in einem der Ansprüche 1 bis 12 definiert, wobei die lokale Fettablagerung mit einem adipösen Zustand, wie beispielsweise Fettleibigkeit, Fettumverteilungssyndrom, Augenlidfettherniation, Lipome, Lipodystrophie (einschließlich Buffalo-Hump-Lipodystrophie), Dorsocervicalfett, viscerale Adipositas, Brustvergrößerung, Hyperadipositas, diffuses Körperfett an Rumpf und Armen, Dercum-Krankheit, Madelung-Nacken, Lipödem, piezogene Knötchen, Launois-Cleret-Syndrom und Xanthelasma, in Verbindung steht.

14. Pharmazeutische Lösung zur Verwendung, wie in einem der Ansprüche 1 bis 13 definiert, wobei die Lösung einem Körperbereich, ausgewählt aus: unterm Auge, unterm Kinn, unterm Arm, Gesäß, Wange, Braue, Wade, Rücken, Oberschenkel, Knöchel und Bauch, verabreicht wird.

## Revendications

1. Solution pharmaceutique pour utilisation thérapeutique dans le retrait non chirurgical d'un ou plusieurs dépôts de graisse localisés dans un sujet, dans laquelle la solution doit être administrée par injection sous-cutanée, ladite solution consistant essentiellement de :
entre 0,05 et 1 % en poids d'un sel biliaire ; et
un excipient pharmaceutique ;
dans laquelle la solution est sans phosphatidylcholine ou phospholipides.

2. Solution pharmaceutique pour utilisation telle que définie dans la revendication 1, dans laquelle ledit sel biliaire est le sel d'un acide biliaire sélectionné à partir du groupe constitué par : l'acide désoxycholique, l'acide cholique, l'acide chénodésoxycholique, l'acide chénodésoxycholique 7-alpha-déshydroxylate, l'acide lithocholique, l'acide ursodésoxycholique, l'acide dihydroxytaurine, l'acide trihydroxytaurine, et des conjugués de glycine de l'un quelconque ci-dessus.

3. Solution pharmaceutique pour utilisation telle que définie dans la revendication 1 ou la revendication 2, dans laquelle ledit sel biliaire comprend un cation sélectionné à partir du groupe constitué par le sodium (Na⁺), le potassium (K⁺), le lithium (Li⁺), le magnésium (Mg²⁺), le calcium (Ca²⁺), le baryum (Ba²⁺), le strontium (Sr²⁺), et l'ammonium (NH₄⁺).

4. Solution pharmaceutique pour utilisation telle que définie dans l'une quelconque des revendications 1 à 3, dans laquelle ledit sel biliaire comprend un métal alcalin sélectionné à partir du groupe constitué par le sodium (Na⁺), le potassium (K⁺), et le lithium (Li⁺).

5. Solution pharmaceutique pour utilisation telle que définie dans l'une quelconque des revendications 1 à 3, dans laquelle ledit sel biliaire comprend un métal alcalinoterreux sélectionné à partir du groupe constitué par le magnésium (Mg²⁺), le calcium (Ca²⁺), le baryum (Ba²⁺), et le strontium (Sr²⁺).

6. Solution pharmaceutique pour utilisation telle que définie dans l'une quelconque des revendications 2 à 5, dans laquelle ledit sel biliaire est l'acide désoxycholique.

7. Solution pharmaceutique pour utilisation telle que définie dans l'une quelconque des revendications 1 à 4, dans laquelle ledit sel biliaire est le désoxycholate de sodium.

8. Solution pharmaceutique pour utilisation telle que définie dans l'une quelconque des revendications 1 à 7, dans laquelle ladite solution est une solution aqueuse.

9. Solution pharmaceutique pour utilisation telle que définie dans la revendication 7 ou la revendication 8, dans laquelle la solution comprend du désoxycholate de sodium à une concentration entre 0,05 % et 0,5 %.

10. Solution pharmaceutique pour utilisation telle que définie dans l'une quelconque des revendications 7 à 9, dans laquelle la solution comprend du désoxycholate de sodium à une concentration entre 0,1 % et 0,5 %, telle que 0,1 % ou 0,5 %.

11. Solution pharmaceutique pour utilisation telle que définie dans l'une quelconque des revendications 1 à 10, dans laquelle la solution comprend en outre un véhicule aqueux sélectionné à partir d'une solution saline, de l'eau, de l'alcool benzylique et de l'acide acétique.

12. Solution pharmaceutique pour utilisation telle que définie dans l'une quelconque des revendications 1 à 11, dans laquelle le dépôt de graisse localisé est dans un humain.

13. Solution pharmaceutique pour utilisation telle que définie dans l'une quelconque des revendications 1 à 12, dans laquelle le dépôt de graisse localisé est associé avec des affections adipeuses telles que, par exemple, l'obésité, le syndrome de redistribution de graisse, la hernie graisseuse de paupière, les lipomes, la lipodystrophie (y compris la lipodystrophie de la bosse de bison), la graisse dorsocervicale, l'adiposité viscérale, l'hypertrophie mammaire, l'hyperadiposité, la graisse corporelle diffuse autour du tronc et des bras, la maladie de Dercum, le collier de Madelung, le lipoedème, les papules piézogènes, le syndrome de launois-cleret et le xanthélasma.

14. Solution pharmaceutique pour utilisation telle que définie dans l'une quelconque des revendications 1 à 13, dans laquelle la solution est délivrée à une zone du corps sélectionnée parmi : sous l'oeil, sous le menton, sous le bras, fesse, joue, front, mollet, dos, cuisse, cheville ou ventre.
